# EUROPEAN PATENT SPECIFICATION

(11) **EP 0 643 834 B1**
(45) Date of publication and mention of the grant of the patent: **16.08.2000**
(21) Application number: 92920466.7
(22) Date of filing: 14.09.1992
(51) Int. Cl.: G01N 33/543, G01N 33/553, G01N 33/94, A61B 5/117

(54) **SIMULTANEOUS DRUG TESTING AND FINGERPRINTING ASSAY**
GLEICHZEITIGES TESTEN VON ARZNEIMITTELN UND FINGERABDRUCKTESTVERFAHREN
ANALYSE SIMULTANEE DE TEST ANTI-DOPAGE ET DE PRISE D'EMPREINTE DIGITALE

(30) Priority: 13.09.1991 US 759922
(43) Date of publication of application: 22.03.1995
(73) Proprietor: LA MINA LTD., Rockville, Maryland 20852 (US)
(72) Inventor: GUIRGUIS, Raouf, A., Rockville, MD 20852 (US)
(74) Representative: McCall, John Douglas
(86) International application number: PCT/US92/07785
(87) International publication number: WO 93/06486

(56) References cited:
- EP-A- 0 440 350
- US-A- 3 419 000
- US-A- 4 810 630
- US-A- 4 963 325
- CHEMICAL ABSTRACTS, Volume 108, No. 8, issued 22 February 1988, SASAKI et al., "Ink-free Fingerprint Recording Films", see page 66, column 2, Abstract No. 57581t; & JP,A,62 266 036.

## Description

### TECHNICAL FIELD OF THE INVENTION

The invention is in the field ligand-receptor assays, including immunoassays, used for determining the presence or absence of an analyte in a biological fluid. More particularly, the apparatuses and methods of the invention relate to establishing the identity of a test subject and determining the presence or absence of at least one analyte in a biological fluid using a single test device.

### BACKGROUND OF INVENTION

For many years, those skilled in the art of ligand-receptor assays have sought an effective, inexpensive, and reliable device and method for detecting the presence and/or absence of antigens, antibodies, and the like. The art is replete with such devices.

For many years, those skilled in the art have also sought devices and methods for assuring that the sample being tested actually came from a certain individual. Many schemes, some elaborate, some simple, have been established to verify that the test sample was in fact produced by a particular individual. Similarly, many schemes exist to thwart matching a particular test result with a particular person.

For example, drug testing is now a routine procedure in athletics, prison, and the work place. The protocols involve testing individual fluid samples such as urine or blood to determine the presence of certain antibodies in the fluid sample; a positive result may be an indication of drug use.

A problem which has occurred during such testing is that test fluids may be obtained from persons other than the person to be tested or that test fluids become mixed, lost, or cannot be specifically identified with that person after the test is returned from the laboratory. Also, these assays typically take too long to obtain timely results.

As noted above, the principles which form the basis for this class of detection techniques is a person's immune system, i.e., the inherent capability of a mammal to respond to a foreign molecule, typically a macromolecule. Hereinafter, any molecule which is capable of eliciting such a response will be referred to as an antigen, e.g., an antibody generator. The proteins and protein fragments which are produced in response to the antigen will be referred to as antibodies or immunoglobulins.

EP-A2-440350 discloses a method and device for testing for the presence of substances such as drugs in the blood while simultaneously positively identifying the test subject. The device comprises an absorbent pad and a membrane (preferably a nitro cellulose membrane) mounted to the absorbent pad. The absorbent pad preferably contains a plurality of separated areas provided with different absorbed antibody having a specific receptor site for specific antigens produced by different predetermined drugs and a base area without antibodies.

The method of testing, disclosed in EP-A2-440350, comprises the steps of: (a) puncturing the finger of a test subject to obtain a quantity of blood on the finger, (b) covering the finger with a thin film of blood, (c) placing the finger on a membrane containing antibodies specifically selected to bind antigens produced by the substances being tested for in the blood so that blood is deposited from the finger on the membrane; and (d) adding colour development solution to the membrane to bring out a colour indicating presence or absence of a substance.

According to one embodiment of this invention there is provided an assay device for determining the presence or absence of at least one analyte in a fluid test sample obtained from a test subject while providing specific identification of the test subject, said device comprising a reaction medium, said reaction medium comprising:
(a) at least one reaction zone which comprises a member of a first ligand/receptor pair immobilized thereon, said member selected such that, when the reaction zone is contacted with a test sample suspected of containing a specific analyte, said member provides a means for determining the presence or absence of said analyte, and
(b) at least one control zone which comprises a member of a second ligand/receptor pair immobilized thereon, said member selected such that, when contacted with a signal producing agent, said member will react with said signal producing agent to form a pattern which is capable of establishing the identity of a test subject.

According to another embodiment of this invention there is provided a method for testing for the presence of at least one analyte in a fluid sample obtained form a test subject and positively identifying the test subject which comprises :
(a) obtaining a sample of fluid from the test subject;
(b) applying said fluid sample to an assay device which comprises a reaction medium, said reaction medium comprising
   (1) at least one reaction zone which comprises a member of a first ligand/receptor pair immobilized thereon, said member selected such that, when the reaction zone is contacted with said fluid sample suspected of containing a specific analyte, said member provides a means for determining the presence or absence of said analyte, and
   (2) at least one control zone for establishing identity of said test subject, said control zone comprising a member of a second ligand/receptor pair immobilized thereon, said member selected such that, when contacted with a signal-producing agent, said member wail react with said signal producing agent to form a pattern which establishes the identity of said test subject;
(c) coating a finger or bottom of a foot of the test subject with a signal producing agent and contacting the control zone of the assay device with said coated finger or foot such that said signal-producing agent reacts with said member of a second ligand/receptor pair to form a pattern which establishes the identity of said test subject; and
(d) determining from the reaction zone whether the fluid test sample contains said specific analyte.

According to a further embodiment of this invention there is provided a method for testing for the presence of at least one analyte in a fluid sample obtained from a test subject and positively identifying the test subject which comprises:
(a) obtaining a sample of fluid suspected of containing a specific analyte from the test subject and combining it with anti-analyte antibodies which specifically bind to said analyte so as to form a solution, whereby said anti-analyte antibodies specifically bind to said analyte if present in said fluid sample;
(b) applying said solution to an assay device which comprises a reaction medium, said reaction medium comprising
   (1) at least one reaction zone which comprises antigens immobilized thereon which specifically bind to said anti-analyte antibodies, such that, when the reaction zone is contacted with said solution unbound anti-analyte antibodies in said solution bind to said immobilized antigens;
   (2) at least one control zone for recording the fingerprint or footprint of said test subject, said control zone comprising anti-species antibody immobilized thereon that specifically binds a labelled antibody;
(c) coating a finger or bottom of a foot of the test subject with a labelled antibody that specifically binds to said anti-species antibody and said anti-analyte antibody;
(d) contacting said control zone and said reaction zone of the assay device with said coated finger or foot such that a fingerprint or footprint, respectively, is produced in said control zone and said labelled antibody binds to said anti-analyte antibody bound in said test zone;
   wherein the presence of said labelled antibody in said test zone indicates the absence of said analytes in said body fluid and the absence of said labeled antibody in said test zone indicates the presence of said analytes in said body fluid.

Also according to a further embodiment of this invention there is provided a method for processing a blood sample comprising:
(a) pricking a finger of a test subject to form a droplet of blood;
(b) smearing the blood to coat the finger with a thin film of blood;
(c) applying the finger to a reaction medium of an assay device and allowing the blood to dry, wherein said reaction medium is a porous medium that includes one member of a first ligand/receptor pair, which member specifically binds to a predetermined analyte in the blood sample;
(d) applying a clearing solution to the reaction medium;
(e) determining the presence or absence of at least one analyte in the test sample; and
(f) forming a test subject identifying pattern in a control zone of the reaction medium, wherein said control zone includes one member of a second ligand/receptor pair, which member specifically binds to a signal producing agent to form a pattern which is capable of establishing the identity of the test subject.

Also according to yet a further embodiment of this invention there is provided a kit for processing a sample comprising:
(a) an assay device in accordance with the invention; and
(b) at least one agent for producing a signal in at least one of said reaction zone and said control zone.

In another embodiment of the invention, various segregated areas of the reaction medium include a specific member of an immunological pair (MIP) which bind to or capture a specific analyte, such as a drug or drug metabolite. Different segregated areas may contain MIPs that are specific for different designated analytes, thereby allowing determinations for more than one analyte to be carried out in a single assay.

By use of the present invention there may be provided one or more of the following:
(i) a specifically designed immunoassay device which allows the determination of the presence or absence of an analyte in a sample, as well as specifically identifying of the person providing the sample.
(ii) a method and device for detecting the presence of specific antigens or specific antibodies in a biological fluid.
(iii) means for positively identifying the individual tested.
(iv) an easily handled, disposable testing pad suitable for detecting the presence or absence of an analyte and for identifying the test subject. For example a finger of the test subject may be coated with a labelled ligand and the finger may be pressed on a membrane substrate having a specific immobilized ligand bed to capture an analyte from a biological fluid while simultaneously providing an inkless (specific binding) visible fingerprint of the testee so that positive identification of the fluid donor is irrefutably obtained. The fingerprint of the fluid donor obtained on the membrane may also include information about the drugs present in his body fluids at the time of performing the test. This information can be recorded or stored for positive identification when needed.
(v) a method to assay a test sample for the presence or absence of an analyte while also identifying the subject being tested. Previously such testing has been accomplished by a series of tests which may involve shifting of the fluid being tested to different containers and removal of the fluid from the person being tested to a distant place. Oftentimes, fluid misplacement and/or substitution opened questions as to the chain of custody of the tested fluid.

The methods and devices according to the invention are particularly beneficial in that only a small amount of signal producing agent is required, in contrast to the conventional flow through cassettes; applying pressure while maintaining direct contact on the surface of the test device maintains a localized concentration of reagents, particularly the signal producing agent, which appears to increase the speed of the reaction, e.g., test results are obtained in a matter of seconds versus a matter of minutes or longer; and, in some embodiments, labeling a secondary antibody (as opposed to labeling the primary antibody or the antigen) increases the reaction time of the assay because the antigen's shape is not altered by being bonded to a label.

### DEFINITIONS

1. LIGAND-RECEPTOR ASSAY: any technique involving the detection of the complex formed between a ligand and a substance which binds to the ligand. Preferably, one member of the complex is an analyte. The preferred ligand-receptor assay is an immunoassay. Ligand-receptor assays may be used to determine the presence, absence, quantity, and concentration of ligands in biological fluids. Ligand-receptor assays may be competitive or non-competitive, homogeneous or heterogeneous, direct or indirect, or a combined detection technique, e.g., binding an antibody to a small chemical moiety such as biotin or dinitrophenol (DNP).

It is preferred that during the assay process, substantially all of at least one predetermined ligand or ligand receptor remains in a predetermined position. Any technique for immobilizing a ligand or ligand receptor is included in the scope of the present invention. In a preferred embodiment, a ligand or ligand receptor is bound or immobilized on or in a solid phase. Typical immobilization mechanisms include, but are not limited to, covalent binding, noncovalent binding, chemical coupling, physical entrapment, and adsorption.

2. LIGAND: refers to the analyte iteself, or a substance which can be used to infer the presence of an analyte in a test sample. A ligand-receptor refers to the substances for which there is a specific binding partner, e.g., the ligand. As used herein, ligand and ligand-receptors may be members of an immunological pair (MIP). Ligands and ligand-receptors may include haptens, hormones, antigens, antibodies (including anti-antibodies and antibody fragments such as Fab or Fc), DNA, RNA, oligonucleotides, nucleic acids, and complexes or metabolites of any of the above. The ligand or the ligand-receptor may be labeled or unlabeled.

3. ANALYTE: the substance to be assayed, and, depending on the specific assay used, may be a ligand or a ligand-receptor. Exemplary analytes include but are not limited to drugs, proteins, haptens, hormones, and other molecules, alone or an combination with a protein. A hapten is a small molecular weight material, such as a some drugs or drug metabolites, which must first attach to a protein in order to be antigenic or immunogenic. For example, some drugs may be found in the body in both free and bound forms, which in turn provides the ability to distinguish between chronic drug abusers and acute drug abusers. 4.BIOLOGICAL FLUID: any body fluid which may be tested to determine the presence or absence of an anlyte, including, but not limited to blood or a blood component, saliva, urine.

5. SIGNAL PRODUCING AGENT: any agent used in a ligand-receptor assay which can be used to produce a detectable signal. The preferred signal producing agents provide an easily visible signal, and more preferably a color.

### BRIEF DESCRIPTION OF THE DRAWINGS

Figure 1 is a plan view of an assay device according to the present invention.
Figure 2 is a plan view of an assay device according to the present invention, illustrating the results of conducting a test for the presence or absence of an antigen and the formation of a pattern suitable for identifying a test subject.
Figure 3 is an enlarged schematic cross sectional view of an assay device according to the present invention, showing a reaction zone in a reaction medium.
Figure 4 is an enlarged schematic cross sectional view of an assay device according to the present invention, showing a control zone in a reaction medium.
Figure 5 is an enlarged schematic cross sectional view of an assay device according to the present invention, illustrating a positive test result.
Figure 6 is an enlarged schematic cross sectional view of an assay device according to the present invention, illustrating a negative test result.
Figure 7 is an abstract representation of a cross section of an assay device according to the present invention, illustrating contacting the assay device method according to the invention.
Figure 8 is an abstract representation of a cross section of an immunoassay device according to the present invention, illustrating the formation of a fingerprint in a control zone of the reaction medium.
Figure 9 is an abstract representation of a cross section of an another embodiment of an immunoassay device according to the present invention, illustrating the transfer of a test mixture from a swab to a finger of the person providing the test sample.
Figure 10 is an abstract representation of a cross section of the immunoassay device of Figure 9, illustrating the application of the test mixture to the reaction medium by the finger of the person providing the test sample.

### DETAILED DESCRIPTION OF THE INVENTION

A ligand receptor assay device according to the present invention includes a reaction medium having at least one reaction zone and at least one control zone which is capable of establishing the identity of a test subject. In a preferred embodiment of the invention, the reaction zone includes at least one member of a ligand, ligand-receptor pair, said member being capable of establishing the presence or absence of a substance in a test sample. In another preferred embodiment of the invention, the identity of the test subject is established by incorporating a ligand in at least one control zone to provide for a test subject identifying pattern.

A method in accordance with the invention includes conducting, on or in a portion of a reaction medium, an immunological assay for the presence or absence of an analyte in a test sample, and separately establishing the identity of the test subject who provided the test sample on a portion of the reaction medium.

One embodiment of the invention is directed to an assay device preferably an immunoassay device, comprising a porous support and a reaction medium covering a portion of the porous support. The reaction medium includes at least one reaction zone and at least one control zone, wherein the control zone is capable of providing a pattern suitable for identifying a test subject. In a more preferred embodiment, the control zone is capable of providing a pattern comprising a fingerprint. The reaction zone includes a means for conducting a ligand/antiligand, preferably a means for conducting an assay for the presence or absence of an analyte. In another preferred embodiment, the means for conducting an assay includes a member of an immunological pair.

Another embodiment of the invention is directed to an assay device comprising a reaction medium having at least one reaction zone and at least one control zone including a member of an immunological pair, wherein the control zone is capable of establishing the identity of a test subject. The control zone is preferably capable of providing a pattern comprising a fingerprint.

According to another aspect, the present invention includes a support, preferably a porous support; a reaction medium, which is preferably a porous medium, mounted on the support, wherein said reaction medium has at least one reaction zone and at least one control zone, and wherein at least a portion of the control zone is capable of establishing the identity of the test subject. A preferred embodiment includes a member of an immunological pair positioned in or on the reaction medium. In another preferred embodiment, at least a portion of a control zone includes one member of an immunological pair. In some embodiments of the invention, the reaction medium may be unsupported.

The present invention is also directed to a method for processing a sample comprising contacting a reaction zone of an assay device with a test sample; determining the presence or absence of at least one analyte in the test sample; and forming a test subject identifying pattern in a control zone of the assay device.

The present invention is also directed to a method for determining the presence of absence of an analyte in a sample including conducting an immunoassay for the analyte in a reaction zone of an immunoassay device, whereby the presence or absence of an analyte in a test sample is determined; and establishing the identity of the person providing the test sample in a control zone on the immunoassay device.

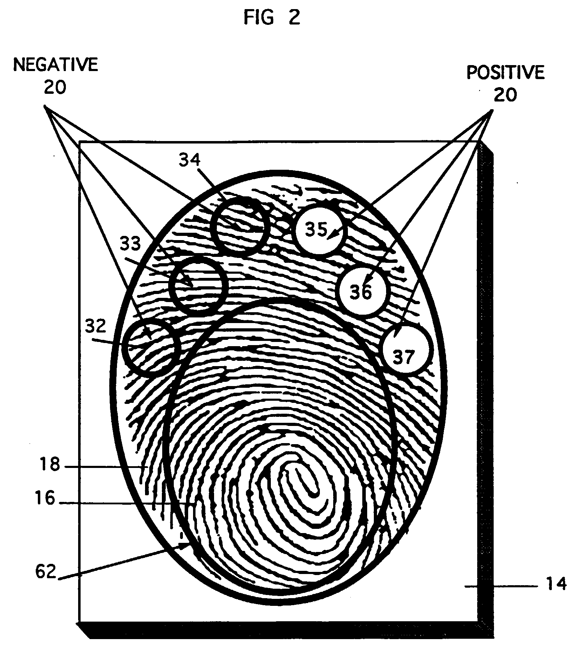

A method according to the invention includes determining the presence or absence of an analyte in a sample, preferably by using any ligand binding based assay, and establishing the identity of the person who provided the sample, preferably by using any immunologically based protocol. The presence and/or absence of an analyte in a sample may be determined by applying a biological fluid to at least one reaction zone 20, and allowing a predetermined ligand/receptor binding reaction to take place. In a preferred embodiment of the invention, completion of the reaction will result in the production of a signal. Preferably, the signal is produced in the form of a visible color or the absence of color. For example, in Figure 2, the production of a visible color hatched areas 32-34 indicates the absence of the analyte in the test sample, and the absence of color areas 35-37 indicates the presence of the analyte in the test sample. It is intended that the invention should not be limited by the specific binding assay employed, the method of producing the signal, the type of signal produced, and the particular location of positive and/or negative results.

In accordance with the invention, the production of an image or pattern which identifies the person providing the test sample may be accomplished by applying the person's finger or foot to a control zone, and allowing a predetermined ligand/receptor reaction to take place, thereby producing the image of a fingerprint. In a preferred embodiment of the invention, the tip of a person's finger, coated with a signal producing agent, preferably a color forming agent, is applied to the surface of a control zone having a MIP which will bond to the signal producing agent coated on the person's finger. In a preferred embodiment of the invention, completion of the immunological reaction will result in the production of a visible color which corresponds to the person's fingerprint. Figure 2 shows an exemplary visible fingerprint.

In an embodiment of the invention, particularly for pediatric use, the image or pattern which may be formed may be of the foot or a portion of the foot. In this embodiment, the foot or a portion of the foot, previously coated with a labeling reagent, may be applied to an apparatus according to the invention.

Exemplary embodiments of the invention will now be described by reference to the Figures.

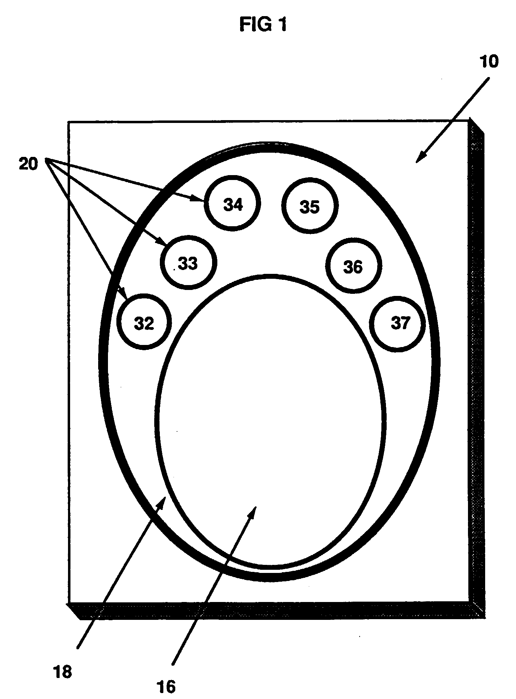

An immunoassay device 10 according to the invention includes a reaction medium 14, preferably mounted on a support or base member 12. Base member 12 is preferably a porous support. In a preferred embodiment of the invention, the reaction medium 14 includes at least one reaction zone 20 and at least one control zone 16. In the exemplary embodiments shown in Figures 1 and 2, the immunoassay device 10 includes six reaction zones 20 (numbered 32-37) and two control zones 16 and 18.

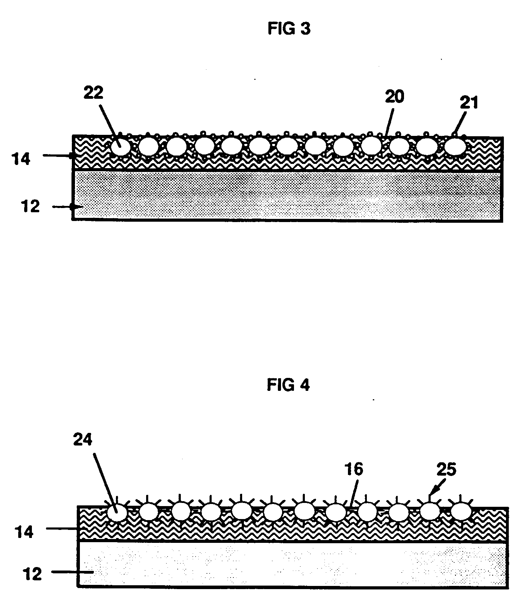

In a detection apparatus 10 according to the invention, at least one of the reaction zones 20 include means for conducting an immunological assay, preferably to establish the presence and/or absence of an analyte in a test sample. In the exemplary embodiment shown in Figure 3, reaction zones 20 include at least one carrier, such as particles 22, embedded in reaction medium 14. As shown, the particles 22 are coated with a member of an immunological pair 21.

In a detection apparatus 10 according to the invention, at least one of the control zones 16 include means for establishing the identity of a test subject, preferably including a member of an immunological pair. In the exemplary embodiment shown in Figure 4, control zone 16 includes a carrier, such as particles 24, embedded in the reaction medium 14. As shown, the particles 24 are coated with a member of an immunological pair 25. In a preferred embodiment of the invention, MIP 25 is capable of bonding to a signal producing agent, or to a MIP-signal producing agent conjugate.

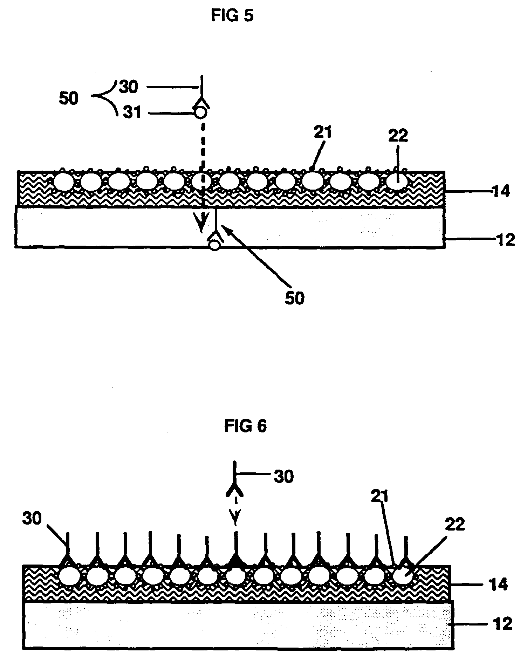

An exemplary method according to the invention may be described by reference to Figures 5-8. In Figure 5, the presence of an analyte 31 in a test sample can be determined. The biological fluid is applied to the surface of reaction zone 20 having particles 22 coated with a member of an immunological pair 21. If analyte 31 is present in the biological fluid, it will bond to or be captured by MIP 30, which is specific for analyte 31. The resulting immunological pair 50 will not bond to or be captured by MIP 21, and will pass through the reaction medium 14. According to this embodiment of the invention, no color forming agent will bond to the coated particles.

In Figure 6, the absence of an analyte in a test sample can be determined. The biological fluid is applied to the surface of reaction zone 20 having particles 22 coated with a member of an immunological pair 21. In the absence of analyte in the biological fluid, MIP 30 will bind or be captured by MIP 21. According to this embodiment of the invention, a color forming agent applied to the surface of the reaction medium will bond to MIPs 30 on the coated particles. In an alternative embodiment of the invention, MIP 30 may include the color forming agent.

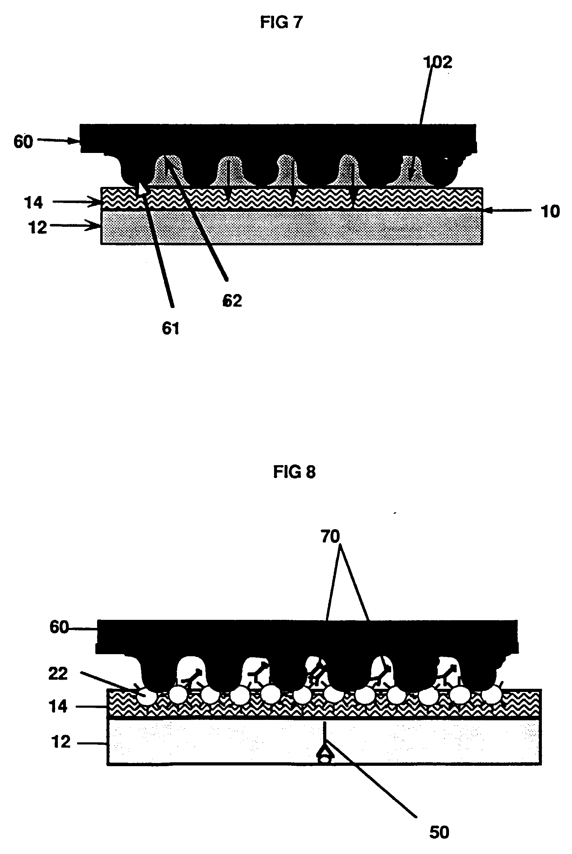

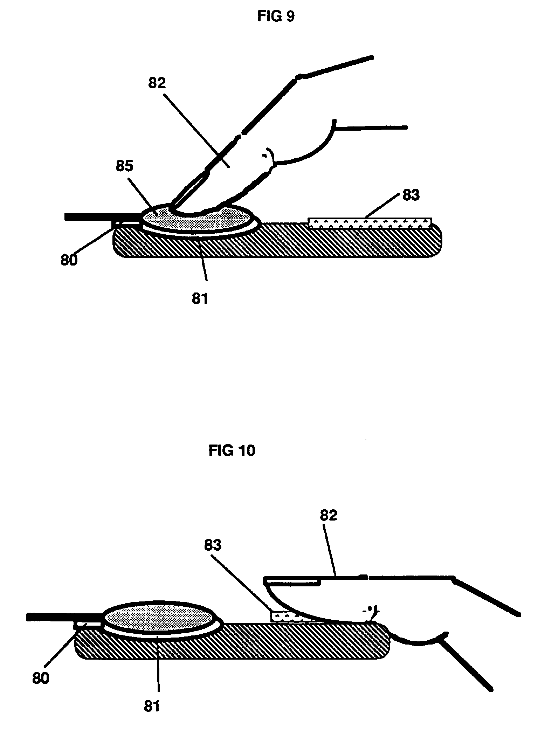

Figures 7 and 8 illustrate an exemplary method of producing the image of a fingerprint on the surface of a control zone. The surface 60 of a person's finger includes ridges 61 and valleys 62. When a person's finger is coated with a color forming reagent 102, a greater portion of the color forming reagent will tend to collect in the valleys 62. When the finger is applied to the surface of the reaction medium 14, it is believed that zones of higher concentration color forming reagent localize in the area of the valleys. The coated particles in the surface of the control zone will typically bond to a higher proportion of the color forming reagent, which will in turn produce a more intense visible color corresponding to the valley of the person's fingerprint.

In a preferred embodiment of the invention, determining the presence/absence of a substance in a biological fluid and producing the image of a fingerprint are typically accomplished in only a few minutes.

Individual aspects of the invention will now be described in greater detail.

### LIGAND-RECEPTOR ASSAY

In accordance with the invention, the detection device may be used with any assay technique for detecting an analyte of interest. In all of the assays used in accordance with the present invention, an analyte is bound to at least one ligand or ligand-receptor. Exemplary binding assays include but are not limited to labeled ligand assays, including competitive binding assays in which a solid phase is coupled to the binding reagent, such as simultaneous or sequential incubation with one or more separation steps, and displacement assays; and labeled binding reagent assays, including noncompetitive and competitive binding assays, including assays in which the solid phase is the binding reagent or the ligand, and sandwich assays.
precipitation, radioimmunoassay, or enzyme-linked immunosorbent assay. It is intended that the invention is not to be limited by the type of immunoassay employed or the specific protocol used in performing the assay. Exemplary immunoassays techniques are shown in the Examples.

### REACTION MEDIUM

In accordance with the invention, the reaction medium may be any medium suitable for performing a ligand-receptor assay. As noted above, the reaction medium includes at least one reaction zone and at least one control zone. Typically, ligand-receptor assays may be conducted using a reaction medium in the form of beads, tubes, plates, paddles, a porous or non-porous matrix, or porous or non-porous membranes or filters.

The reaction medium can include a depth medium wherein the surface or the an area near the surface comprises at least one reaction zone. The reaction medium may also include a layered structure having a reaction zone as a surface layer. In accordance with the invention, the reaction and control zones are positioned in the reaction medium so that a signal producing agent, if present, can be readily visualized.

In accordance with the invention, the preferred reaction medium is porous, and even more preferred, a porous membrane. It is intended that the invention should not be limited by the type or construction of reaction medium. One skilled in the art will readily recognize that the choice of reaction medium depends on the desired physical properties, including, but not limited to properties such as sensitivity, binding capacity, stability, the type of molecules or MIPs which can be bound, and compatibility with a particular assay protocol.

Exemplary materials for forming the reaction medium include, but are not limited to latex, nitrocellulose, nylon, and cellulose.

A preferred reaction medium is the commercially available Gelman Supor membrane. Supor membrane is a latex low protein binding polysulfone membrane with a hydrophilic surface. One skilled in the art will recognize that this membrane may be desirable because it provides superior flow rate and particle retention; a smooth surface; brilliant whiteness and opaqueness to enhance signal contrast in diagnostic tests; low extractables to reduce sample contamination and background interference; and uniform porosity to ensure final product consistency. Also, the use of this membrane obviates the need for an external wetting agent, which may be desirable for controlling the introduction of unwanted extractables.

Another preferred membrane, IAM, Immobilon Affinity Membrane (commercially available from Millipore Corporation; see, for example, U.S. Patent 4,407,943), the hydrophobic base polymer, polyvinylidenedifluoride (PVDF), binds proteins. IAN also permits a high degree of control over the extent of protein binding and the user can reproducibly immobilize nanogram to microgram quantities of protein on the surface to suit various assay requirements. Binding a ligand to IAN and its use in immunoassays is known in the art.

The use of membranes, as the solid support in a reaction medium of the present invention may be desirable for the added convenience of a solid phase, the typically high protein binding capacity, and the membrane's flow-through characteristics.

Nitrocellulose is one of the most commonly used membranes due to its high affinity for proteins nucleic acids, other cellular antigens, and cellular macromolecules, and may be desirable if the desired MIP binding is ionic or hydrophobic. Nitrocellulose also provides an excellent matrix for blotting proteins and nucleic acids. The nitrocellulose may be cut into whatever shape is required and has the useful characteristic that the amount protein in a fingerprint will be clearly visible.

Included within the scope of the present invention is changing or incorporating different surface properties on the membrane in order to achieve a desired result, e.g., the surface properties of a membrane designed for a competitive binding assay for a hormone may be different than an immunometric assay for a therapeutic drug. For example, it has been shown that treating the surface of a hydrophilized PVDF membrane with ethanolamine reduces the non-specific binding of the membrane surface. Selection of a particular surface treatment agent or surface property may be based on the desired chemical characteristic imparted to the surface; inability or reduced capability of denaturing or impairing the functionality of a bioactive agent on or in the reaction zone; to affect a certain orientation of an immobilized bioactive molecule; to promote long-term stability of an immobilized bioactive molecule; the inclusion of a desired nucleophilic substituent; and availability and cost. The use of other surface treatment agents, including bifunctional or multifunctional reagents, to affect the surface properties of the membrane are included within the present invention.

One skilled in the art will readily recognize the benefit derived by including a reaction medium which is porous. Positive test samples will readily pass through the reaction medium, obviating the need to wash the surface of the reaction medium prior to adding a color-forming agent. Although it is less desirable, it is intended that the invention include the use of a non-porous medium.

As noted above, the reaction medium may be supported or unsupported. In the preferred embodiment, the reaction medium is supported, and even more preferred, the support is porous. An exemplary support includes, but is not limited to polystyrene.

In another embodiment of the invention, the support may be impermeable to the fluids and reagents used in conducting the testing; in this embodiment, the reaction medium is preferably "deep" enough to have a reaction zone at or near the surface of the medium.

### CONTROL ZONE

In a preferred embodiment of the invention, the reaction medium includes a control zone for establishing a reference point in determining the presence or absence of an analyte in a test sample (reference control zone), and a control zone for establishing the identity of the test subject which provided the test sample (identity control zone).

In accordance with the invention, a control zone for establishing the identity of the test subject (identity control zone) which provided the test sample may include any signaling mechanism which incorporates a MIP. For example, the control zone may include a member of an immunological pair capable of bonding to a signal producing agent, which when applied by contacting the test subject's finger to the surface of the identity control zone, an image of the fingerprint is produced on the test device.

As used herein, capable of establishing the identity of a test subject refers to the capability of specifically identifying the subject who provided the sample being tested. In a preferred embodiment of the invention, the control zone includes a member of an immunological pair which, when placed in contact with the test subject's finger which has been previously coated with a signal producing agent, is capable of producing an image of a fingerprint, preferably a fingerprint image easily visible. In a less desirable embodiment, the invention includes a control zone having a member of an immunological pair or nucleotide sequences which are capable of bonding to nucleotide sequences which can be used to identify the test subject.

In accordance with the invention, the control zone for establishing a reference point in determining the presence or absence of an analyte in a test sample includes any means for providing a control. Several examples for providing a control are show in the examples. It is intended that the invention is not to be limited by the means or mechanism for establishing the standard of comparison in judging the test results.

### REACTION ZONE

The reaction zone refers to the locus in which the presence or absence of an analyte can be determined.
For example, the reaction zone is preferably on or near the surface of the reaction medium, or may be below the surface. In a preferred embodiment of the invention, the reaction zone includes at least one ligand or ligand-receptor bound to a particle or bead in a matrix or filter, suitable for capturing an analyte of interest. In another embodiment of the invention, at least one of the reaction zones may be a control for establishing a reference point, e.g., a comparative control to show the absence of color.

### SIGNAL PRODUCING AGENT

A signal producing agent refers to any agent or marker produces a detectable signal or which permits the detection of a ligand. Preferred signal producing agents are those which permit detection of the analyte without instruments, preferably by visual means. Exemplary signal producing agents include, but are not limited to color forming agents, such as an enzyme, polymers containing dyes, chemiluminescent agents, fluorescent agents, radioisotopes or ferromagnetic particles. The color forming agent may be a colored particle, a colored molecule or some species, such as an enzyme, which is capable of triggering a sequence of events leading to the formation of a colored marker. The colored molecule may be a fluorescent dye, such as fluorescein or rhodamine; a chemiluminescent compound; a bioluminescent compound; or a compound that may be detected by the absorption of electromagnetic radiation, including ultraviolet radiation, visible radiation and infrared radiation. The colored molecule may be directly or indirectly conjugated to a ligand or ligand-receptor. Alternatively, the colored molecule may be incorporated in a particle, particularly a microsome.

Enzymes, useful as color forming agents, include alkaline phosphatase, horseradish peroxidase or B-galactosidase. Such enzymes are often used in conjunction with a chromogenic substrate. A list of some exemplary chromogenic substrates which may be used with enzyme color forming agents are given below in Example 11.

In a preferred embodiment of the invention the color forming agent may be an electron dense particle, such as colloidal gold, silver coated colloidal gold or ferritin.

In another preferred embodiment of the invention, the color forming agent is capable of bonding to a MIP.

The present invention includes signal producing agent, which may be a color forming agent. Preferably, the signal producing agent comprises a labeled MIP, for detecting the adsorption of an analyte or an analyte conjugate in or on the reaction medium. The labeled MIP may comprise any one of a number of appropriate labels, such as an enzyme, a fluorescent compound, a bioluminescent compound, a ferromagnetic atom or a colored particle or microsome. In a preferred embodiment, the label used in the present invention is colloidal gold. Gold is biologically inert, has very good charge distribution and is widely available in many useful forms. Its detection can be enhanced using several silver deposition methods, which permit development to be monitored by the naked eye. Colloidal gold particles conjugated with a wide range of anti-immunoglobulin antibodies, protein A or streptavidin are available commercially.

While colloidal gold substrate is preferred over other dyed particles or microsomes, a chromogenic substrate provides an alternate sensitive detection method for the enzyme conjugate.

### METHOD

A method in accordance with the invention includes conducting a ligand-receptor assay for an analyte in a reaction zone of an assay device, whereby the presence or absence of an analyte in the test sample is determined; and establishing the identity of the person providing the test sample in a control zone on the assay device. In a preferred embodiment of the invention, establishing the identity of the person providing the test sample may be accomplished using ligand-receptor assay methods, e.g., binding a MIP to a signal producing agent.

As noted above, the biological fluid sample to be tested for the presence or absence of at least one analyte is applied to the surface of the immunoassay device according to the invention. Any means of applying the fluid sample to the detection apparatus may be used. For example, in one embodiment of the invention, the biological fluid, e.g., urine, may be combined with a MIP according to standard immunoassay procedures, drawn into a pipette, and deposited on the surface of the test device.

In another example, the biological fluid, e.g., blood, may be collected in a pipette and deposited on the surface of the test device, or may be smeared on a solid applicator, such as a fingertip. In a preferred embodiment, blood is smeared on the test subject's fingertip, which is then pressed in contact with the surface of the detection device. Other exemplary methods of applying the test sample to the detection device are shown in the Examples.

A preferred embodiment of the invention includes applying one or more reagents to a surface of the test device using pressure. For example, it has been found that smearing a signal-producing agent on the test subject's fingertip and applying the fingertip to the test device significantly reduces the amount of signal-producing agent required, and significantly increases the speed at which the assay proceeds. For example, as little as 10 microliters and preferably about 12 microliters of colloidal gold can be smeared on the test subject's fingertip in order to perform the assay. In contrast, as much as 100 microliters or more of the signal producing agent is typically required for flow through cassette assay devices.

While not intending to be limited to a particular theory of operation, it is believed that the pressure and/or surface tension which occurs when the test subject's finger is placed on the assay device results in localizing an increased concentration of ligand and ligand receptor, increasing the amount of time that the reagents are retained in a localized area of the assay device, and may involve migration of the signal producing agent from the ridges of the fingerprint to the valleys, to produce a clear fingerprint pattern on the surface of the test device.

The test device preferably includes one or more MIPs immobilized in a reaction zone. For example, if the analyte of interest is an antibody, the test device may include an antigen bound to microspheres or particles embedded in the reaction zone. One skilled in the art will recognize that a desired amount of antigen can be immobilized in the reaction zone, and that a threshold concentration of bound MIP may be used to detect a predetermined amount of analyte. In one embodiment of the invention, the detection device includes several reaction zones, each reaction zone for a different analyte, and each reaction zone includes a predetermined threshold concentration of bound MIP to detect a predetermined amount of analyte. As used herein, threshold amount or concentration refers to the lower limit of a concentration range for an analyte. In another embodiment of the invention the detection device includes several reaction zones, each reaction zone including a different threshold concentration for the same analyte. In this embodiment, the threshold concentration provides a reference point for determining the upper limit of an analyte concentration range. As shown in the Examples, varying the threshold concentration in the reaction zones may an exemplary method of quantifying the amount of analyte in a sample.

After the biological fluid being tested has been applied to the surface of the detection device, the presence or absence of the analyte in the test sample can be determined, preferably by visually ascertaining a color (signal) or the absence of a color (signal). In a preferred embodiment of the invention, a positive response, i.e., indicating the presence of the analyte in the biological fluid, corresponds to the absence of color. In a preferred embodiment of the invention, a negative response, i.e., indicating the absence of the analyte in the biological fluid, corresponds to a visualized color.

In accordance with one aspect of the invention, when the biological fluid being tested is blood, it has been unexpectedly found that whole blood may be directly applied to the surface of the test device without the need for serum separation prior to the sample being applied to the test device. In accordance with this embodiment of the invention, the whole blood sample is applied to the surface of the test device, and then a detergent is applied over the blood sample. While it is known that detergents such as Tween may be used to remove red cells and the like from whole blood samples, it has been unexpectedly found that the application of a clearing reagent, such as a detergent (preferably a detergent which includes a mixture of ionic, nonionic, and alcohol), allows the application of whole blood to the surface of the assay device without the need to separate the cellular component from the serum prior to application to the device. The application of a detergent is preferable because the detergent disrupts blood cells. The ruptured cell debris then pass through the medium and are removed from the reaction zone, i.e., removed from interfering with immobilized surface antigens.

Once the presence or absence of the analyte has been determined, the detection device made in accordance with the present invention may be used to identify the test subject whose biological fluid was tested. In a preferred embodiment of the invention, determining the identity of the test subject includes performing a ligand-receptor assay which results in the development of a fingerprint of the individual. In accordance with a preferred embodiment of the invention, determining the identity of the test subject is independent of any reactions with the analyte in the fluid.

In accordance with this method of the invention, the person's fingertip is coated with a signal producing agent conjugated to a MIP. When the coated fingertip is applied to the surface of a control zone, the signal producing conjugate binds to a MIP embedded in the control zone of the test device. While the invention is not to be limited to a particular theory of operation, it is believed that the ridges and valleys of a person's fingertip provide localized areas of concentrated signal producing conjugate (corresponding to a valleys) and localized areas of low concentration signal producing agent (corresponding to a ridge). When applied to the surface of a control zone of a device according to the invention, a clearly identifiable image of a fingerprint is produced, whereby the identity of the person providing the test sample can be provided.

In accordance with the invention, the presence or absence of an analyte of interest in a sample may be determined by applying the sample to the surface of a reaction medium, said reaction medium including at least one reaction zone having a member of an immunological pair therein; and applying a signal producing agent which binds, directly or indirectly (e.g., a secondary antibody), to the MIP or to the analyte of interest.

### TEST KIT

In an embdoiment of the invention, the assay device made in accordance with the invention may be incorporated into a kit. The kit may also include any of a number of reagents for performing the ligand receptor assays. For example, the kit may include a test device, at least one agent for producing a signal (or an "ink" pad for transferring the signal producing agent to the finger), and at least one washing, clearing or detergent reagent. The kit may also include one or more ligands or ligand receptors, such as lyophilzied primary antibody and a secondary antibody, and one or more buffers.

### SPECIFIC EXAMPLES

### EXAMPLE 1.

One embodiment of a immunoassay device of the present invention was prepared according to the following procedure. A reaction zone was constructed by embedding polystyrene particles, coated with human serum albumin-benzoylecgonine (HSA-BE), in one portion of a low protein binding polysulfone membrane (Gelman Supor membrane) mounted on a porous support. A control zone 16 was constructed by embedding polystyrene particles, coated with goat anti-mouse antibody, in a different portion of the membrane. A representative schematic cross section of the reaction and control zones is shown in Figures 3 and 4.

The assay device was prepared according to the following procedure. The reaction medium was first rinsed with 400 to 500 of a blocking buffer solution (Solution A) consisting of 2% polyvinyl alcohol (PVA), 1% glycine and 0.05% Tween-20 in a phosphate buffered saline solution. The central well of the immunoassay device (control zone 16) was spotted with a dilute solution of polystyrene latex coated with goat anti-mouse immunoglobulin G (adsorbed against human serum) in a phosphate buffered saline containing 4% sucrose, 1.0% BSA and 0.05% azide. The reaction zone was spotted with a dilute solution of polystyrene latex coated with human serum albumin-benzoylecgonine (HSA-BE) in 0.2 M sodium bicarbonate. The reaction medium was then inverted onto hydrophobic polyethylene and dried for one hour in a drying room set between 80 and 100°F. After removal of the reaction medium from the hydrophobic polyethylene, the assay device was ready for use.

### EXAMPLE 2.

A saliva sample was obtained from a person to be tested for the presence or absence of benzoylecgonine. A 200 microliter aliquot of a dilute solution of mouse anti-benzoylecgonine immunoglobulin G (mouse anti-BE IgG) in a phosphate buffered saline, which contained 0.1% bovine serum albumin (BSA) and 0.05% Tween-20, was added to a 200 microliter aliquot of the saliva sample. The resulting mixture was allowed to incubate for three minutes. During this incubation period, 400 microliters of Solution A was added to the reaction medium of the immunoassay device prepared in Example 1 and allowed to drain through the reaction medium. The mouse anti-BE IgG/saliva mixture was then added to the reaction medium and allowed to incubate for two minutes. Another 400 microliter aliquot of Solution A was then added to the reaction medium and allowed to drain. After the Solution A had finished draining, a finger of the person who had provided the saliva sample was painted with 15 microliters of a dilute solution of colloidal gold conjugated goat anti-mouse immunoglobulin G in a TRIS buffer containing 1.0% BSA, 0.05% Tween-20 and 0.05% azide (hereinafter "Gold Label Solution"). The finger was gently pressed against the reaction medium of the immunoassay device and held in place for three seconds. The finger was then carefully rolled off the reaction medium. The reaction medium was allowed to incubate for fifteen seconds before another 400 microliter aliquot of Solution A was added to the reaction medium. After completion of the procedure, the reaction zone was colored, showing a negative test result for benzoylecgonine (e.g., as shown with reaction zones 32-34 in Figure 2).

The determination was repeated except that a minor amount of benzoylecgonine was added to the saliva sample just prior to the addition of the mouse anti-BE IgG solution. In this instance, after completion of the test, the reaction zones were completely white (e.g., as shown with reaction zones 35-37 in Figure 2). At the completion of both determinations, the central well control zone contained an imprint of the finger of the person who had provided the saliva sample (e.g., as shown with the fingerprint 62 on control zone 16 in Figure 2).

### EXAMPLE 3.

An immunoassay device is prepared according to the procedure of Example 1 except that the device contains six reaction zones for different analytes. Each reaction zone contains polystyrene particles coated with a different analyte conjugate embedded in the membrane. Reaction zones 32-37 contain polystyrene latexes coated respectively with cocaine (32), opiates (33), PCP (34), amphetamine/methamphetamine (35), tetrahydrocannabinol (36) and alcohol (37) as the analytes (as shown in Figure 1). The central control zone 16 is prepared as in Example 1 with polystyrene particles, coated with goat anti-mouse immunoglobulin G, embedded in the membrane.

A saliva sample is obtained from a person to be tested for the presence or absence of the six analytes. A 200 microliter aliquot of a dilute solution of mouse anti-analyte immunoglobulin G for each of the six different analytes in a phosphate buffered saline (hereinafter "mouse anti-analyte IgG Solution I") is added to 200 microliters of the saliva sample. As described in Example 2, while this mixture is incubating, the reaction medium is pretreated with Solution A. After the mouse anti-analyte IgG Solution I/saliva mixture is added to the reaction medium, the remainder of the test procedure described in Example 2 is followed. The reaction medium is treated with Solution A, contacted with a finger of the test subject that has been coated with a dilute solution of colloidal gold conjugated goat anti-mouse immunoglobulin G and then treated again with Solution A. After this procedure is carried out, central control zone 16 contains the imprint of the finger of the person providing the urine sample. Reaction zones 32-37 are all colored, showing a negative test result for the presence of all six analytes.

The remaining saliva is then spiked with minor amounts of amphetamine/methamphetamine, tetrahydrocannabinol and alcohol. A 200 microliter aliquot of mouse anti-analyte IgG Solution I is then added to 200 microliters of the spiked saliva and the determination is repeated as described for the unspiked sample. After the procedure is carried out, the assay device exhibits the result shown in Figure 2. Central control zone 16 contains the imprint of the finger of the person providing the urine sample. Reaction zones 32-34 show a negative test result and are colored. Reaction zones 35-37 show a positive result and are white.

### EXAMPLE 4.

An immunoassay device, containing six reaction zones, is prepared according to the procedure of Example 1. As in Example 3, reaction zones 32-37 respectively contain polystyrene latexes coated with cocaine (32), opiates (33), PCP (34), amphetamine/methamphetamine (35), tetrahydrocannabinol (36) and alcohol (37) as the analytes. In this instance, however, the central control zone 16, prepared as in Example 1, contains polystyrene particles coated with mouse anti-goat immunoglobulin G embedded in the membrane. A urine sample is obtained from a person to be tested for the presence or absence of the six analytes. A 200 microliter aliquot of the urine is mixed with 200 microliters of mouse anti-analyte IgG Solution I and the test procedure is then carried out exactly as described in Example 2. After completion of the determination, the central control zone 16 contains the imprint of the finger of the person providing the saliva sample and reaction zones 32-37 are all colored, showing a negative test result for the presence of all six analytes.

The remaining urine is then spiked with minor amounts of amphetamine/methamphetamine, tetrahydrocannabinol and alcohol. A 200 microliter aliquot of mouse anti-analyte IgG Solution I is then added to 200 microliters of the spiked urine and the determination is repeated as described for the unspiked sample. After completion of the determination, the immunoassay device exhibits the result shown in Figure 2. Central control zone 16 contains the imprint of the finger of the person providing the saliva sample. Reaction zones 32-34 are colored (negative result) and reaction zones 35-37 are white (positive result).

### EXAMPLE 5.

The reaction medium 83 of the immunoassay device shown in Figures 9 and 10 is prepared according to the procedure described in Example 1. The reaction medium 83 has six reaction zones 32-37, which contain polystyrene latexes coated respectively with cocaine (32), opiates (33), PCP (34), methamphetamine (35), tetrahydrocannabinol (36) and alcohol (37) as the analytes and a central control zone 16 containing polystyrene particles, coated with mouse anti-goat immunoglobulin G, embedded in the membrane (as shown in Figures 1).

A sterile swab 85 is placed under the tongue of a person whose saliva is to be tested for the presence or absence of the six analytes. After one minute, the swab 85 is removed and is placed in the incubation channel 80 and basin 81 of the immunoassay device (as shown in Figure 9). Three drops of a dilute solution of silver coated gold microsome conjugates of mouse anti-analyte immunoglobulin G for each of the six different analytes (hereinafter "mouse anti-analyte IgG Solution II") are added to the saliva swab. The treated swab is allowed to incubate for one minute before a finger 82 of the person providing the saliva sample is pressed against the swab for ten seconds. The finger is then lifted off the swab, immediately pressed on the reaction medium of the immunoassay device and held in place for thirty seconds. The finger is then lifted off the reaction medium and the medium is allowed to incubate for two minutes before the result is read. At this, point the central control zone 16 contains the imprint of the finger of the person providing the saliva sample and reaction zones 32-37 are all colored, showing a negative test result for the presence of all six analytes.

The test procedure is repeated using a new sterile swab except that prior to the addition of the mouse anti-analyte IgG Solution II to the swab, a drop of a solution containing minor amounts of methamphetamine, tetrahydrocannabinol and alcohol is added to the swab. After completion of the test, the immunoassay device exhibits the result shown in Figure 2. Central control zone 16 contains the imprint of the finger of the person providing the saliva sample. Reaction zones 32-34 are colored (negative result) and reaction zones 35-37 are white (positive result).

### EXAMPLE 6.

An immunoassay device is prepared as in Example 5. A saliva sample is collected from under the tongue of a person to be tested for the presence or absence of the six analytes using a sterile swab. While the saliva sample is being collected, 800 microliters of mouse anti-analyte IgG Solution I is placed in a sample tube together with one drop of a dilute solution of PCP. The saliva-containing swab is allowed to soak in the solution in the sample tube for two minutes. A 400 microliter aliquot of the resulting mixture is applied to the reaction medium and allowed to incubate for twenty seconds. A 400 microliter aliquot of Solution A was then added to the reaction medium and allowed to drain. After the Solution A had finished draining, a finger of the person who had provided the saliva sample is painted with 15 microliters of Gold Label Solution. The finger is immediately pressed gently against the reaction medium of the immunoassay device and held in place for five seconds. After the finger is carefully rolled off the reaction medium, the medium is allowed to incubate for fifteen seconds before another 400 microliter aliquot of Solution A is added to the reaction medium. After the completion of the procedure, the reaction zone is completely white, showing a positive test result for PCP. The central control zone contains an imprint of the finger of the person who had provided the saliva sample.

### EXAMPLE 7.

An immunoassay device is prepared according to the procedure of Example 1. The reaction medium comprises a nylon membrane. The reaction zone includes polystyrene particles coated with human serum albumin-cocaine embedded in the membrane. The central control zone includes polystyrene particles, coated with goat anti-human immunoglobulin G, embedded in the membrane.

A finger of a person to be tested for the presence or absence of cocaine in their blood is pricked with a needle to obtain a droplet of blood. The droplet is spread with a sterile spatula to form a thin film of blood over the fingertip. The blood covered fingertip is pressed against the reaction medium and held in place for about ten seconds. After the finger is lifted off, the reaction medium is treated with a dilute solution of household detergent, thereby removing the red blood color from the reaction medium. After the red blood color has been removed, the reaction medium is treated with a dilute solution of alkaline phosphatase conjugated mouse anti-cocaine immunoglobulin G and allowed to incubate for two minutes. The reaction medium is then treated sequentially with Solution A, a dilute solution of 5-bromo-4-chloro-3-indolyl phosphate and nitroblue tetrazolium (BCIP/NBT) and again with Solution A. The reaction medium is allowed to incubate for several minutes between the BCIP/NBT treatment and the final Solution A treatment. When the procedure is completed, the central control zone contained an imprint of the finger of the person who had provided the blood sample and the reaction zone was colored, showing a negative test result for cocaine.

### EXAMPLE 8.

An immunoassay device is prepared as in Example 7. A 500 microliter aliquot of a dilute solution of mouse anti-cocaine immunoglobulin G in a phosphate buffered saline containing 0.1% bovine serum albumin (BSA) and 0.05% Tween-20 (hereinafter "mouse anti-cocaine IgG Solution"), is placed in a small sample tube. One drop of a solution containing a minor amount of cocaine is added to the sample tube.

A finger of a person to be tested for the presence
or absence of cocaine in their blood is pricked with a needle to obtain a droplet of blood and two droplets of blood are added to the mixture in the sample tube. After the resulting mixture is allowed to incubate for two minutes in the sample tube, the mixture is added to the reaction medium of the immunoassay device. The mixture is incubated for one minute on the reaction medium, which is then rinsed with Solution A. A finger of the person who had provided the blood sample is painted with 15 microliters of Gold Label Solution. The finger is gently pressed against the reaction medium of the immunoassay device, held in place for five seconds and then carefully rolled off the reaction medium. The reaction medium is allowed to incubate for fifteen seconds before another 400 microliter aliquot of Solution A is added to the reaction medium. After completion of the procedure, the reaction zone is completely white (positive test for cocaine) and the control zone contains the imprint of the finger of the person providing the blood sample.

### EXAMPLE 9.

An immunoassay device is prepared according to the procedure of Example 1 except that the device contains six reaction zones (see Figure 1). Each reaction zone contains polystyrene particles coated with a different amount of cholesterol conjugate embedded in the membrane. Reaction zone 32 contains a cholesterol conjugate which will produce a positive signal when contacted with a biological fluid having greater than or equal to 150 milligram/deciliter total cholesterol level. Reaction zones 33-36 contain intermediate amounts of cholesterol conjugate, wherein the amount of cholesterol conjugate increases sequentially from zone 32 to zone 35, i.e., 180 mg/dl, 240 mg/dl, 280 mg/dl. The reaction medium also includes two reference control zones corresponding to 30 ml/dl and 65 ml/dl high density lipoprotein.

A finger of a person to be tested for the amount of cholesterol in their blood is pricked with a needle to obtain a droplet of blood. The droplet is added to a small sample tube containing 400 microliters of a dilute solution of mouse anti-cholesterol immunoglobulin G and colloidal gold conjugated mouse anti-cholesterol immunoglobulin G in a phosphate buffered saline containing 0.1% bovine serum albumin (BSA) and 0.05% Tween-20 (hereinafter "mouse anti-cholesterol IgG Solution"). After incubation for three minutes, a thin film of the mixture is spread over a fingertip of the person providing the blood sample using a sterile spatula. The fingertip is immediately gently pressed against the reaction medium of the immunoassay device. After holding the finger in place for five seconds, the finger is carefully lifted off the reaction medium. The reaction medium is incubated for one minute and then treated with a dilute solution of household detergent to remove red blood color from the reaction medium. After completion of the procedure, the control zone contains the imprint of the finger of the person providing the blood sample. Reaction zones 32-33 are colored (negative test for cholesterol), while reaction zones 34 and 35 are white (positive test for cholesterol). Each reaction zone gives a positive test for cholesterol when at least the predetermined threshold amount of cholesterol is present. The threshold cholesterol concentration differs for each reaction zone in correspondence to the amount of cholesterol conjugate adsorbed in the reaction medium in that zone.

### EXAMPLE 10.

A listing of a number of other drugs, the presence or absence of which may be determined using the present invention follows:

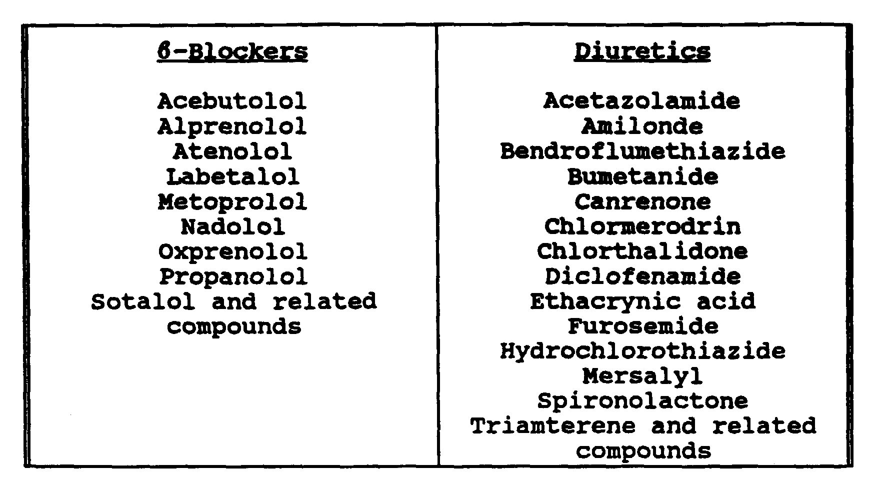

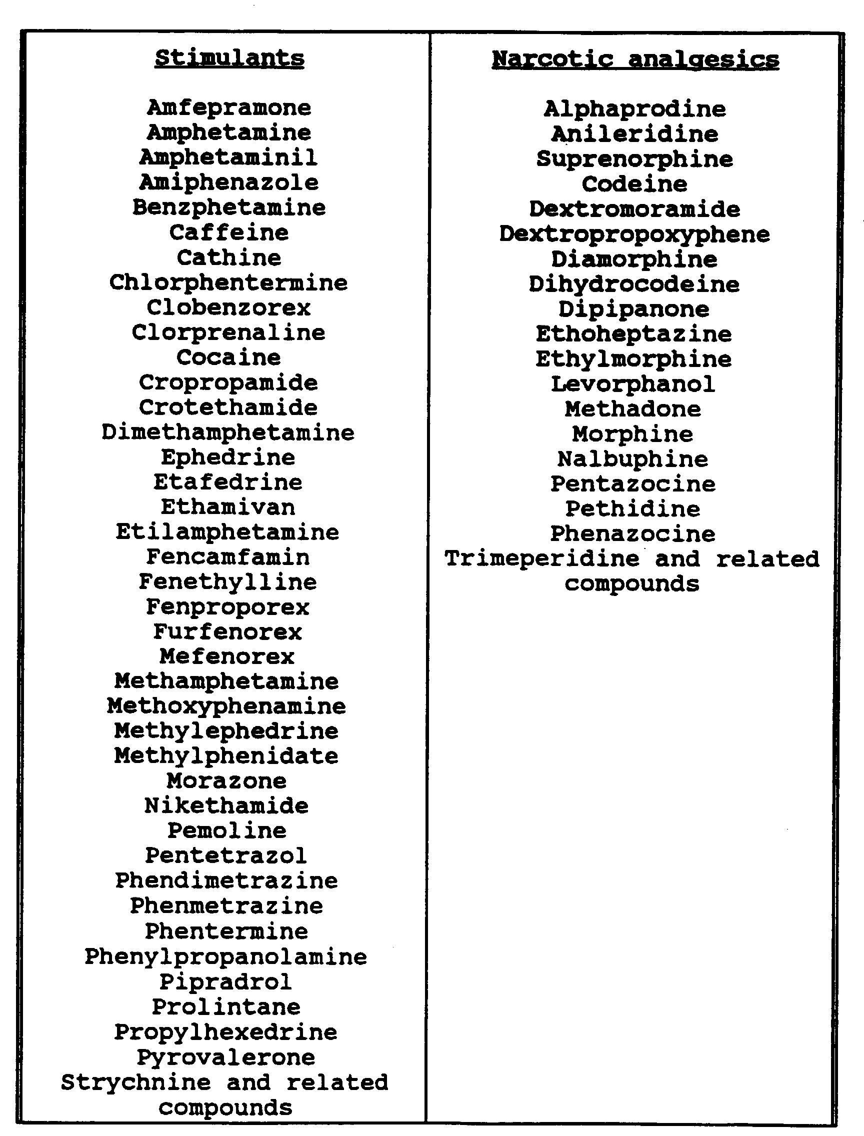

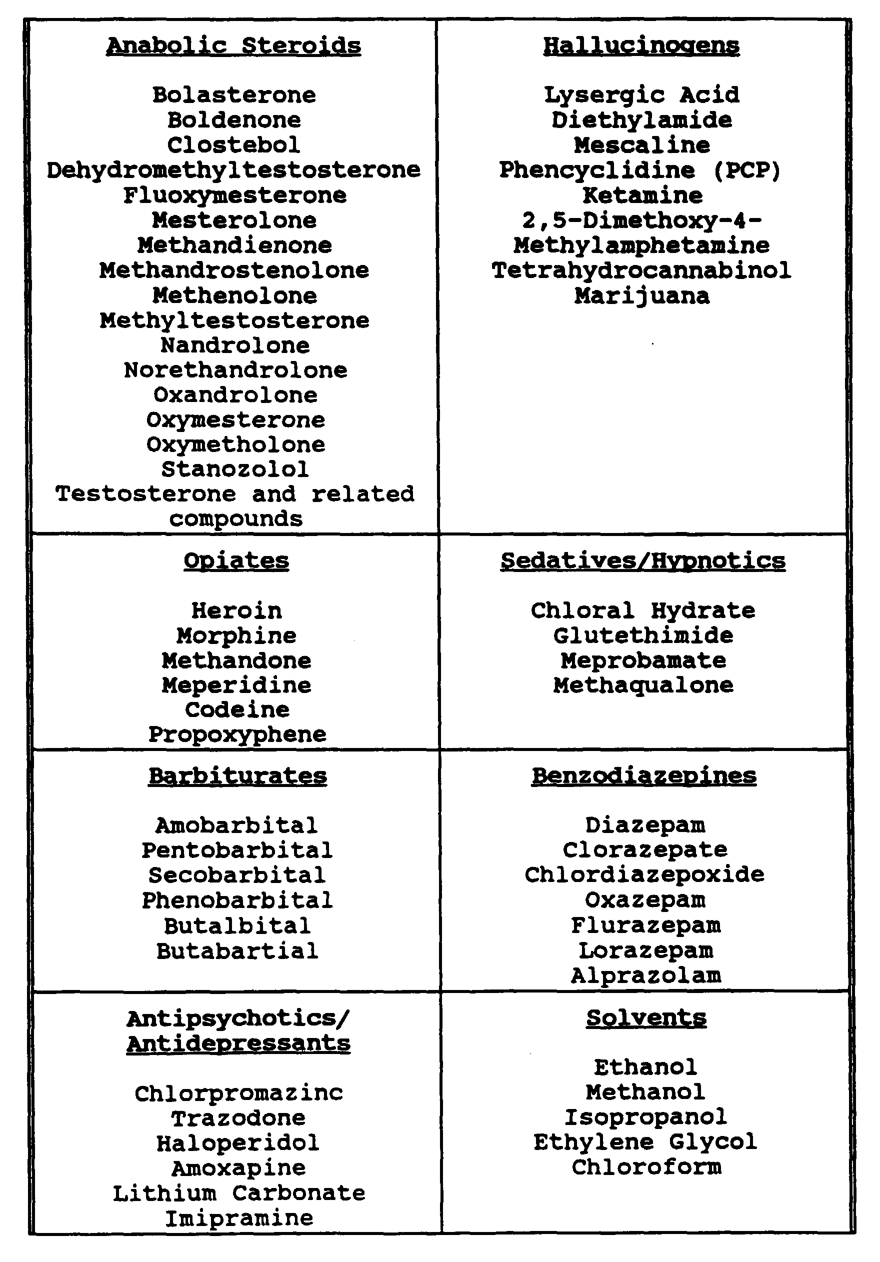

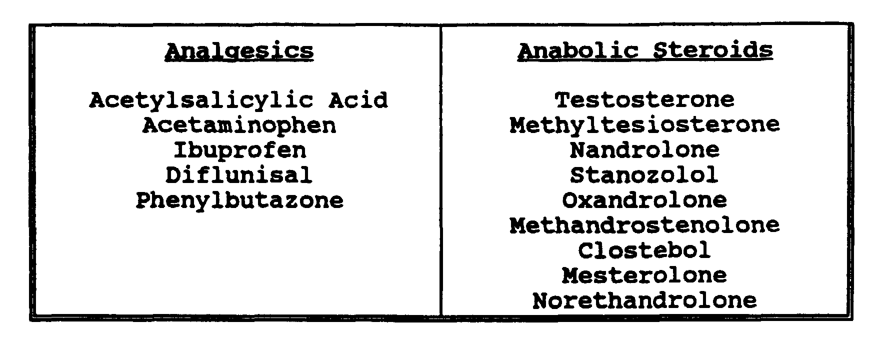

### EXAMPLE 11.

The following table sets forth exemplary chromogenic substrates yielding water-insoluble products that may be used with an appropriate enzyme conjugate in the invention, instead of the colloidal gold label previously noted. Labeling methods utilizing enzyme/chromogen couples are well known and would be easily practiced by one skilled in the art.

| Chromogenic Substrates Yielding Water-Insoluble Products | | | | |
|---|---|---|---|---|
| Final Enzyme Color | Substrate | Abbreviation | Orig. Color | |
| Horseradish Brown Peroxidase | Diaminobenzidene | DAB | Clear | |
| Grey/Black | Diaminobenzidene with nickel enhancement | DAB/nickel | Clear | |
| | 3-Amino-9-ethylcarbazole | AEC | Clear | Red |
| Blue | 4-Chloro-1-naphthol | --- | Clear | |
| Alkaline Phosphatase | Naphthol-AS-BI-phosphate/fast red TR | NABP/FR | Clear | Red |
| | Naphthol-AS-MX-phosphate/fast red TR | NAMP/FR | Clear | Red |
| | Naphthol-AS-BI-phosphate/new fuchsin | NABP/NF | Clear | Red |
| Clear | Bromochloroindolyl Purple phosphate/nitro-blue tetrazolium | NBT | BCIP/ | |
| Blue | 5-Bromo-4-chloro-3-indolyl-B-d-galactopyranoside | BCIG | Clear | |
| B-Galactosidase | Naphthol AS-BI-B-d-galactopyranoside | NABG | Clear | Red |

### EXAMPLE 12.

In this Example, the device and method of Example 7 were used, except that the assay device included reaction zones for the analytes, shown in Example 3, the surface of the reaction medium was pre-wetted with Solution A, the test subject's finger was pricked to produce a sufficient quantity of blood to smear the blood over the tip of the subject's finger, and the smeared blood was allowed to dry on the subject's finger. The finger with the dried blood was then applied to surface of assay device and held in contact with the device for about 15 seconds. After the finger is lifted off, the reaction medium was treated with a dilute solution of clearing reagent, thereby removing the red blood color from the reaction medium. After the red blood color was removed, the reaction medium is treated with a dilute solution of conjugates of colloidal gold bonded to a secondary antibody, each conjugate specifically binding to one of the named analytes. After incubation for about two minutes, a fingertip which had not been used to apply the blood was coated with about 10 microliters of colloidal gold conjugated to a ligand specific for a ligand receptor in the control zone. After the fingertip was removed, the central control zone contained an imprint of the finger of the person who had provided the blood sample and the reaction zone was colored, showing a negative test result for the analytes.

### EXAMPLE 13.

In this Example, the device and method of Example 7 were used, except that the assay device included reaction zones for the analytes, shown in Example 3, the surface of the reaction medium was dry, the test subject's finger was pricked to produce a sufficient quantity of blood to smear the blood over the tip of the subject's finger, and the smeared blood was immediately (before drying) applied to the reaction medium for about 15 seconds. After the finger is lifted off, the reaction medium was allowed to dry (typically about 1 to 2 minutes), treated with a clearing solution to remove the red blood cells, and treated with a dilute solution of clearing reagent, thereby removing the red blood color from the reaction medium. After the red blood color was removed, the reaction medium is treated with a dilute solution of conjugates of colloidal gold bonded to a secondary antibody, each conjugate specifically binding to one of the named analytes. After incubation for about two minutes, a fingertip which had not been used to apply the blood was coated with about 10 microliters of colloidal gold conjugated to a ligand specific for a ligand receptor in the control zone. After the fingertip was removed, the central control zone contained an imprint of the finger of the person who had provided the blood sample and the reaction zone was colored, showing a negative test result for the analytes.

### EXAMPLE 14.

An assay device, containing five reaction zones (corresponding to areas 32 and 34-37 in Figure 1), is prepared according to the procedure of Example 1. As in Example 3, the reaction zones contain polystyrene latexes coated respectively with cocaine (32), PCP (34), amphetamine/ methamphetamine (35), tetrahydrocannabinol (36) and alcohol (37) as the analytes. In this instance, however, zone 33 does not contain any adsorbed analyte or antibody and serves as a reference control zone, i.e., a zone which will remain completely white at the conclusion of the test. The central control zone ("identity control zone" corresponding to zone 16 in Figure 1) is prepared as in Example 1, except that it contains polystyrene particles coated with goat anti-rabbit immunoglobulin G embedded in the membrane.

The reaction medium is treated with 500 microliters of Solution A, thereby wetting the reaction medium. A sample of blood is obtained from a test subject to test for the presence or absence of the five analytes in their blood. A portion of the sample is coated on the test subject's fingertip and applied to the five reaction zones and the reference control zone. Pressure is maintained on the reaction medium for about 10-15 seconds.

A few drops, e.g., about a 500 microliter aliquot of a dilute household detergent solution is then added to the reaction medium and allowed to drain. The household detergent treatment lyses any red blood cells present in the reaction medium and rinses the dark red color from the blood out of the medium. The five reaction zones and the reference control zone are then treated with about 10 ml of a dilute solution of a mixture of colloidal gold conjugated mouse anti-analyte immunoglobulin G in a TRIS buffer containing 1.0% BSA, 0.05% Tween-20 and 0.05% azide.

A finger of the person who had provided the blood sample is then painted with 15 microliters of a dilute solution of colloidal gold conjugated rabbit anti-goat immunoglobulin G in a TRIS buffer containing 1.0% BSA, 0.05% Tween-20 and 0.05% azide. The finger is gently pressed against the identity control zone of the reaction medium and held in place for three seconds. After the finger is carefully lifted off the reaction medium, the medium is allowed to incubate for fifteen seconds before another 400 microliter aliquot of Solution A is added to the reaction medium.

After completion of the procedure, the reaction zones 32 and 34-37 are colored, showing a negative test result for the analytes. The reference control zone 33 is completely white and the identity control zone contains the imprint of the finger of the person providing the blood sample.

Although the present invention has been described in terms of an exemplary embodiment, it is not limited to this embodiment. Alternative embodiments, examples, and modifications which would still be encompassed by the invention may be made by those skilled in the art, particularly in light of the foregoing teachings. Therefore, the invention covers any examples, modifications or alternative embodiments, examples, modifications or equivalents falling within the scope of the claims.

## Claims

1. An assay device for determining the presence or absence of at least one analyte in a fluid test sample obtained from a test subject while providing specific identification of the test subject, said device comprising a reaction medium, said reaction medium comprising:
(a) at least one reaction zone which comprises a member of a first ligand/receptor pair immobilized thereon, said member selected such that, when the reaction zone is contacted with a test sample suspected of containing a specific analyte, said member provides a means for determining the presence or absence of said analyte, and
(b) at least one control zone which comprises a member of a second ligand/receptor pair immobilized thereon, said member selected such that, when contacted with a signal producing agent, said member will react with said signal producing agent to form a pattern which is capable of establishing the identity of a test subject.

2. An assay device as claimed in claim 1, wherein the control zone is capable of providing a pattern for identifying the test subject, wherein the pattern comprises a fingerprint.

3. An assay device as claimed in claim 1, wherein the control zone is capable of providing a pattern for identifying the test subject, wherein the pattern comprises a footprint.

4. An assay device as claimed in any one of the preceding claims, wherein said one member of a ligand receptor pair is capable of determining the presence or absence of at least one of cocaine, benzoylecgonine, opiates, phencyclidine, amphetamine, methamphetamine, tetrahydrocannabinol and alcohol.

5. An assay device as claimed in any one of the preceding claims, wherein the reaction zone includes a member of an immunological pair.

6. An assay device as claimed in any one of the preceding claims, wherein said member of said second ligand/receptor pair is a member of an immunological pair.

7. An assay device as claimed in any one of the preceding claims, wherein the reaction medium is a porous membrane.

8. An assay device as claimed in any one of the preceding claims, wherein the reaction medium comprises more than one reaction zone.

9. An assay device as claimed in any one of the preceding claims, wherein the signal producing agent comprises a member of a ligand/receptor pair labeled with a color forming agent.

10. An assay device as claimed in any one of the preceding claims, wherein the signal producing agent comprises a color forming agent.

11. An assay device as claimed in any one of the preceding claims, wherein the test sample is a biological fluid.

12. An assay device as claimed in claim 11, wherein the biological fluid is blood, a blood product, saliva or urine.

13. An assay device as claimed in any one of the preceding claims, wherein said member of said first ligand/receptor pair is antigen or antibody coated particles.

14. An assay device as claimed in any one of the preceding claims, wherein said member of said first ligand/receptor pair is antibody coated particles.

15. An assay device as claimed in any one of the preceding claims, which includes:
a porous support;
said reaction medium covering a portion of the porous support.

16. An assay device as claimed in any one of the preceding claims, wherein the presence of an analyte produces a color and the absence of an analyte does not produce a color.

17. A method for testing for the presence of at least one analyte in a fluid sample obtained form a test subject and positively identifying the test subject which comprises :
(a) obtaining a sample of fluid from the test subject;
(b) applying said fluid sample to an assay device which comprises a reaction medium, said reaction medium comprising
(1) at least one reaction zone which comprises a member of a first ligand/receptor pair immobilized thereon, said member selected such that, when the reaction zone is contacted with said fluid sample suspected of containing a specific analyte, said member provides a means for determining the presence or absence of said analyte, and
(2) at least one control zone for establishing identity of said test subject, said control zone comprising a member of a second ligand/receptor pair immobilized thereon, said member selected such that, when contacted with a signal-producing agent, said member with react with said signal producing agent to form a pattern which establishes the identity of said test subject;
(c) coating a finger or bottom of a foot of the test subject with a signal producing agent and contacting the control zone of the assay device with said coated finger or foot such that said signal-producing agent reacts with said member of a second ligand/receptor pair to form a pattern which establishes the identity of said test subject; and
(d) determining from the reaction zone whether the fluid test sample contains said specific analyte.

18. A method as claimed in claim 17, wherein contacting the reaction medium with the test sample comprises applying the test sample to the reaction medium with an applicator.

19. A method as claimed in claim 18, wherein the applicator comprises a finger, a foot, a swab or a pipette.

20. A method as claimed in any one of claims 17 to 19, wherein forming the test subject identifying pattern comprises applying a finger of the test subject to the control zone, thereby producing a fingerprint.

21. A method as claimed in any one of claims 17 to 20, wherein establishing the identity of the person further comprises coating the finger of the person with a signal producing agent prior to applying the finger to the control zone.

22. A method as claimed in claim 21, wherein the signal producing agent comprises a member of a ligand/receptor pair labeled with a coloring forming agent.

23. A method as claimed in claim 21, wherein the signal producing agent comprises a color forming agent.

24. A method as claimed in claim 23, wherein the coloring forming agent comprises colloidal gold.

25. A method as claimed in claim 24, further comprising contacting the control zone with a silver coating solution subsequent to applying the finger to the control zone.

26. A method as claimed in any one of claims 17 to 25, wherein the analyte is at least one of cocaine, benzoylecgonine, opiates, phencyclidine, amphetamine, methamphetamine, tetrahydrocannabinol or alcohol.

27. A method as claimed in any one of claims 17 to 26, wherein conducting the ligand/receptor assay includes determining the presence or absence of at least one analyte in a biological fluid.

28. A method as claimed in claim 27, wherein the biological fluid is blood, a blood product, saliva or urine.

29. A method as claimed in any one of claims 17 to 28, wherein conducting the ligand/receptor assay comprises applying blood to the reaction medium and subsequently applying a clearing solution to the reaction medium.

30. A method as claimed in any one of claims 17 to 29, wherein conducting the ligand/receptor assay comprises determining the presence or absence of a threshold amount of at least one analyte in the test sample.

31. A method as claimed in any one of claims 17 to 30, wherein determining the presence of the analyte produces a color in a reaction zone of the reaction medium and determining the absence of the analyte does not produce a color in the reaction zone.

32. A method as claimed in any one of claims 17 to 31, wherein determining the presence of the analyte produces a color in a reaction zone of the reaction medium and determining the absence of the analyte does not produce a color in the reaction zone.

33. A method as claimed in any one of claims 17 to 32, wherein step c includes directly contacting the control zone with a labeled applicator.

34. A method as claimed in any one of claims 17 to 33, wherein said member of a first ligand/receptor pair comprises antigen or antibody-coated particles.

35. A method as claimed in claim 34, wherein said fluid sample is combined with anti-analyte antibodies prior to being applied to said reaction medium, and said member of a first ligand/receptor pair comprises antigens which specifically bind with said anti-analyte antibodies.

36. A method as claimed in any one of claims 17 to 33, wherein said member of a first ligand/receptor pair comprises anti-analyte antibodies.

37. A method for testing for the presence of at least one analyte in a fluid sample obtained from a test subject and positively identifying the test subject which comprises:
(a) obtaining a sample of fluid suspected of containing a specific analyte from the test subject and combining it with anti-analyte antibodies which specifically bind to said analyte so as to form a solution, whereby said anti-analyte antibodies specifically bind to said analyte if present in said fluid sample;
(b) applying said solution to an assay device which comprises a reaction medium, said reaction medium comprising
(1) at least one reaction zone which comprises antigens immobilized thereon which specifically bind to said anti-analyte antibodies, such that, when the reaction zone is contacted with said solution unbound anti-analyte antibodies in said solution bind to said immobilized antigens;
(2) at least one control zone for recording the fingerprint or footprint of said test subject, said control zone comprising anti-species antibody immobilized thereon that specifically binds a labelled antibody;
(c) coating a finger or bottom of a foot of the test subject with a labelled antibody that specifically binds to said anti-species antibody and said anti-analyte antibody;
(d) contacting said control zone and said reaction zone of the assay device with said coated finger or foot such that a fingerprint or footprint, respectively, is produced in said control zone and said labelled antibody binds to said anti-analyte antibody bound in said test zone;
wherein the presence of said labelled antibody in said test zone indicates the absence of said analytes in said body fluid and the absence of said labelled antibody in said test zone indicates the presence of said analytes in said body fluid.

38. A method for processing a blood sample comprising:
(a) pricking a finger of a test subject to form a droplet of blood;
(b) smearing the blood to coat the finger with a thin film of blood;
(c) applying the finger to a reaction medium of an assay device and allowing the blood to dry, wherein said reaction medium is a porous medium that includes one member of a first ligand/receptor pair, which member specifically binds to a predetermined analyte in the blood sample;
(d) applying a clearing solution to the reaction medium;
(e) determining the presence or absence of at least one analyte in the test sample; and
(f) forming a test subject identifying pattern in a control zone of the reaction medium, wherein said control zone includes one member of a second ligand/receptor pair, which member specifically binds to a signal producing agent to form a pattern which is capable of establishing the identity of the test subject.

39. A method for processing a blood sample as claimed in claim 38, which additionally comprises applying a clearing solution to the reaction medium prior to applying the finger.

40. A kit for processing a sample comprising:
(a) an assay device as claimed in any one of claims 1 to 16; and
(b) at least one agent for producing a signal in at least one of said reaction zone and said control zone.

41. A kit as claimed in claim 40, wherein the signal producing agent is conjugated to a member of a ligand/receptor pair.

42. A kit as claimed in claim 40 or 41, further comprising a sample collection device.

## Patentansprüche

1. Analysevorrichtung zur Bestimmung der Anwesenheit oder der Abwesenheit wenigstens eines Analyten in einer Testfluidprobe, die von einer Testperson erhalten wurde, bei der spezifische Identifizierung der Testperson geliefert wird, wobei die genannte Vorrichtung ein Reaktionsmedium umfaßt und das genannte Reaktionsmedium aufweist :
(a) wenigstens eine Reaktionszone, die ein Element eines darauf immobilisierten ersten Paars von Ligand/Rezeptor aufweist, wobei das genannte Element so ausgewählt ist, daß, wenn die Reaktionszone mit einer Testprobe in Kontakt gebracht wird, von der der Verdacht besteht, daß sie einen speziellen Analyten enthält, das genannte Element ein Mittel zum Bestimmen der Anwesenheit oder Abwesenheit des genannten Analyten bereitstellt, und
(b) wenigstens eine Kontrollzone, die ein Element eines darauf immobilisierten Paars von Ligand/Rezeptor aufweist, wobei das genannte Element so ausgewählt ist, daß, wenn es mit einem Signale erzeugenden Mittel in Kontakt gebracht wird, das genannte Element mit dem genannten Signale erzeugenden Mittel zur Bildung eines Musters reagieren wird, das die Identität einer Testperson nachweisen kann.

2. Analysevorrichtung nach Anspruch 1, bei der die Kontrollzone ein Muster zum Identifizieren der Testperson bereitstellen kann, wobei das Muster einen Fingerabdruck aufweist.

3. Analysevorrichtung nach Anspruch 1, bei der die Kontrollzone ein Muster zum Identifizieren der Testperson bereitstellen kann, wobei das Muster einen Fußabdruck aufweist.

4. Analysevorrichtung nach einem der vorhergehenden Ansprüche, bei dem das genannte eine Element eines Paars von Ligand/Rezeptor die Anwesenheit oder Abwesenheit wenigstens eines Stoffes von Kokain, Benzoylecgonin, Opiaten, Phencyclidin, Amphetamin, Methamphetamin, Tetrahydrocannabinol und Alkohol bestimmen kann.

5. Analysevorrichtung nach einem der vorhergehenden Ansprüche, bei der die Reaktionszone ein Element eines immunologischen Paars umfaßt.

6. Analysevorrichtung nach einem der vorhergehenden Ansprüche, bei der das genannte Element des genannten zweiten Paars von Ligand/Rezeptor ein Element eines immunologischen Paars ist.

7. Analysevorrichtung nach einem der vorhergehenden Ansprüche, bei der das Reaktionsmedium eine poröse Membran darstellt.

8. Analysevorrichtung nach einem der vorhergehenden Ansprüche, bei der das Reaktionsmedium mehr als eine Reaktionszone aufweist.

9. Analysevorrichtung nach einem der vorhergehenden Ansprüche, bei der das Signale erzeugende Mittel ein Element eines Paars von Ligand/Rezeptor aufweist, das mit einem Farbbildungsmittel markiert ist.

10. Analysevorrichtung nach einem der vorhergehenden Ansprüche, bei der das Signale erzeugende Mittel ein Farbbildungsmittel aufweist.

11. Analysevorrichtung nach einem der vorhergehenden Ansprüche, bei der die Testprobe ein biologisches Fluid darstellt.

12. Analysevorrichtung nach Anspruch 11, bei der das biologische Fluid Blut, ein Blutprodukt, Speichel oder Urin darstellt.

13. Analysevorrichtung nach einem der vorhergehenden Ansprüche, bei der das genannte Element des genannten ersten Paars von Ligand/Rezeptor mit Antigen oder Antikörper beschichtete Partikeln darstellt.

14. Analysevorrichtung nach einem der vorhergehenden Ansprüche, bei der das genannte Element des genannten ersten Paars von Ligand/Rezeptor mit Antikörper beschichtete Partikeln darstellt.

15. Analysevorrichtung nach einem der vorhergehenden Ansprüche, die umfaßt:
einen porösen Träger;
wobei das genannte Reaktionsmedium einen Teil des porösen Trägers bedeckt.

16. Analysevorrichtung nach einem der vorhergehenden Ansprüche, bei der die Anwesenheit eines Analyten eine Farbe erzeugt und die Abwesenheit eines Analyten keine Farbe erzeugt.

17. Verfahren zum Prüfen hinsichtlich der Anwesenheit wenigstens eines Analyten in einer von einer Testperson erhaltenen Fluidprobe und zum positiven Identifizieren der Testperson, das umfaßt:
(a) eine Fluidprobe von der Testperson zu erhalten;
(b) die genannte Fluidprobe einer Analysevorrichtung zuzuführen, die ein Reaktionsmedium aufweist, wobei das genannte Reaktionsmedium umfaßt:
(1) wenigstens eine Reaktionszone, die ein Element eines darauf immobilisierten ersten Paars von Ligand/Rezeptor aufweist, wobei das genannte Element so ausgewählt ist, daß, wenn die Reaktionszone mit der genannten Fluidprobe in Kontakt gebracht wird, von der der Verdacht besteht, daß sie einen speziellen Analyten enthält, das genannte Element ein Mittel zum Bestimmen der Anwesenheit oder Abwesenheit des genannten Analyten bereitstellt, und
(2) wenigstens eine Kontrollzone zum Nachweisen der Identität der genannten Testperson, wobei die genannte Kontrollzone ein Element eines darauf immobilisierten zweiten Paars von Ligand/Rezeptor aufweist und das genannte Element so ausgewählt wird, daß, wenn es mit einem Signale erzeugenden Mittel in Kontakt gebracht wird, das genannte Element mit dem genannten Signale erzeugenden Mittel zum Bilden eines Musters reagieren wird, das die Identität der genannten Testperson nachweist;
(c) einen Finger oder eine Fußsohle der Testperson mit einem Signale erzeugenden Mittel zu beschichten und die Kontrollzone der Analysevorrichtung mit dem genannten beschichteten Finger oder Fuß so in Kontakt zu bringen, daß das genannte Signale erzeugende Mittel mit dem genannten Element eines zweiten Paars von Ligand/Rezeptor zum Bilden eines Musters reagiert, das die Identität der genannten Testperson nachweist; und
(d) von der Reaktionszone zu bestimmen, ob die Fluidtestprobe den genannten speziellen Analyten enthält.

18. Verfahren nach Anspruch 17, bei dem das in Kontakt Bringen des Reaktionsmediums mit der Testprobe umfaßt, die Testprobe mit einem Applikator dem Reaktionsmedium zuzuführen.

19. Verfahren nach Anspruch 18, bei dem der Applikator einen Finger, einen Fuß, einen Tupfer oder eine Pipette aufweist.

20. Verfahren nach einem der Ansprüche 17 bis 19, bei dem die Bildung des die Testperson identifizierenden Musters umfaßt, einen Finger der Testperson auf die Kontrollzone aufzulegen, wodurch ein Fingerabdruck erzeugt wird.

21. Verfahren nach einem der Ansprüche 17 bis 20, bei dem der Nachweis der Identität der Person weiter umfaßt, den Finger der Person mit einem Signale erzeugenden Mittel zu beschichten, bevor der Finger auf die Kontrollzone aufgelegt wird.

22. Verfahren nach Anspruch 21, bei dem das Signale erzeugende Mittel ein Element eines Paars von Ligand/Rezeptor aufweist, das mit einem Farbbildungsmittel markiert ist.

23. Verfahren nach Anspruch 21, bei dem das Signal erzeugende Mittel ein Farbbildungsmittel aufweist.

24. Verfahren nach Anspruch 23, bei dem das Farbbildungsmittel Kolloidgold aufweist.

25. Verfahren nach Anspruch 24, das weiter umfaßt, die Kontrollzone nach Auflegen des Fingers auf die Kontrollzone mit einer Silberbeschichtungslösung in Kontakt zu bringen.

26. Verfahren nach einem der Ansprüche 17 bis 25, bei dem der Analyt wenigstens einen Stoff von Kokain, Benzoylecgonin, Opiaten, Phencyclidin, Amphetamin, Methamphetamin, Tetrahydrocannabinol oder Alkohol darstellt.

27. Verfahren nach einem der Ansprüche 17 bis 26, bei dem die Ligand/Rezeptor-Analyse umfaßt, die Anwesenheit oder Abwesenheit wenigstens eines Analyten in einem biologischen Fluid zu bestimmen.

28. Verfahren nach Anspruch 27, bei dem das biologische Fluid Blut, ein Blutprodukt, Speichel oder Urin darstellt.

29. Verfahren nach einem der Ansprüche 17 bis 28, bei dem die Durchführung der Ligand/Rezeptor-Analyse umfaßt, dem Reaktionsmedium Blut zuzuführen und nachfolgend dem Reaktionsmedium eine Klärlösung zuzuführen.

30. Verfahren nach einem der Ansprüche 17 bis 29, bei dem die Durchführung der Ligand/Rezeptor-Analyse umfaßt, die Anwesenheit oder Abwesenheit einer Grenzmenge wenigstens eines Analyten in der Testprobe zu bestimmen.

31. Verfahren nach einem der Ansprüche 17 bis 30, bei dem. die Bestimmung der Anwesenheit des Analyten eine Farbe in einer Reaktionszone des Reaktionsmediums erzeugt, und die Bestimmung der Abwesenheit des Analyten keine Farbe in der Reaktionszone erzeugt.

32. Verfahren nach einem der Ansprüche 17 bis 31, bei dem die Bestimmung der Anwesenheit des Analyten eine Farbe in einer Reaktionszone des Reaktionsmediums erzeugt und die Bestimmung der Abwesenheit des Analyten keine Farbe in der Reaktionszone erzeugt.

33. Verfahren nach einem der Ansprüche 17 bis 32, bei dem Schritt c umfaßt, die Kontrollzone direkt mit einem markierten Applikator in Kontakt zu bringen.

34. Verfahren nach einem der Ansprüche 17 bis 33, bei dem das genannte Element eines ersten Paars von Ligand/Rezeptor mit Antigen oder Antikörper beschichtete Partikeln umfaßt.

35. Verfahren nach Anspruch 34, bei dem die genannte Fluidprobe mit Anti-Analyt-Antikörpern kombiniert wird, bevor sie dem genannten Reaktionsmedium zugeführt wird, und das genannte Element eines ersten Paars von Ligand/Rezeptor Antigene aufweist, die sich spezifisch an die genannten Anti-Analyt-Antikörper binden.

36. Verfahren nach einem der Ansprüche 17 bis 33, bei dem das genannte Element eines ersten Paars von Ligand/Rezeptor Anti-Analyt-Antikörper aufweist.

37. Verfahren zum Prüfen hinsichtlich der Anwesenheit wenigstens eines Analyten in einer von einer Testperson erhaltenen Fluidprobe und zum positiven Identifizieren der Testperson, das umfaßt:
(a) eine Fluidprobe, bei der der Verdacht besteht, daß sie einen speziellen Analyten enthält, von der Testperson zu erhalten und sie mit Anti-Analyt-Antikörpern zu kombinieren, die sich spezifisch an den genannten Analyten zur Bildung einer Lösung binden, wodurch die genannten Anti-Analyt-Antikörper spezifisch an den genannten Analyten gebunden werden, wenn dieser in der genannten Fluidprobe vorhanden ist;
(b) die genannte Lösung einer Analysevorrichtung zuzuführen, die ein Reaktionsmedium aufweist, wobei das genannte Reaktionsmedium umfaßt
(1) wenigstens eine Reaktionszone, die darauf immobilisierte Antigene aufweist, welche sich spezifisch an die genannten Anti-Analyt-Antikörper binden, so daß, wenn die Reaktionszone mit der genannten Lösung in Kontakt gebracht wird, nicht gebundene Anti-Analyt-Antikörper in der genannten Lösung sich an die genannten immobilisierten Antigene binden;
(2) wenigstens eine Kontrollzone zum Aufzeichnen des Fingerabdrucks oder Fußabdrucks der genannten Testperson, wobei die genannte Kontrollzone einen darauf immobilisierten Anti-Spezies-Antikörper aufweist, der sich spezifisch an einen markierten Antikörper bindet;
(c) einen Finger oder eine Fußsohle der Testperson mit einem markierten Antikörper zu beschichten, der sich spezifisch an den genannten Anti-Spezies-Antikörper und den genannten Anti-Analyt-Antikörper bindet;
(d) die genannte Kontrollzone und die genannte Reaktionszone der Analysevorrichtung mit dem genannten beschichteten Finger oder Fuß so in Kontakt zu bringen, daß ein Fingerabdruck bzw. Fußabdruck in der genannten Kontrollzone erzeugt wird und der genannte markierte Antikörper sich an den in der genannten Testzone gebundenen genannten Anti-Analyt-Antikörper bindet;
wobei die Anwesenheit des genannten markierten Antikörpers in der genannten Testzone die Abwesenheit der genannten Analyten in dem genannten Körperfluid anzeigt und die Abwesenheit des genannten markierten Antikörpers in der genannten Testzone die Anwesenheit der genannten Analyten in dem genannten Körperfluid anzeigt.

38. Verfahren zum Verarbeiten einer Blutprobe, umfassend:
(a) einen Finger einer Testperson zum Bilden eines Bluttropfens anzustechen;
(b) das Blut zum Überziehen des Fingers mit einer dünnen Blutschicht zu verschmieren;
(c) den Finger auf ein Reaktionsmedium einer Analysevorrichtung aufzulegen und das Blut trocknen zu lassen, wobei das genannte Reaktionsmedium ein poröses Medium ist, das ein Element eines ersten Paars von Ligand/Rezeptor einschließt, welches Element sich spezifisch an einen vorbestimmten Analyten in der Blutprobe bindet;
(d) eine Klärlösung dem Reaktionsmedium zuzuführen;
(e) die Anwesenheit oder Abwesenheit wenigstens eines Analyten in der Testprobe zu bestimmen; und
(f) ein eine Testperson identifizierendes Muster in einer Kontrollzone des Reaktionsmediums zu bilden, wobei die genannte Kontrollzone ein Element eines zweiten Paars von Ligand/Rezeptor einschließt, welches Element sich spezifisch an ein Signale erzeugendes Mittel zum Bilden eines Musters bindet, das die Identität der Testperson nachweisen kann.

39. Verfahren zum Verarbeiten einer Blutprobe nach Anspruch 38, das zusätzlich umfaßt, dem Reaktionsmedium vor Auflegen des Fingers eine Klärlösung zuzuführen.

40. Testsatz zum Verarbeiten einer Probe, umfassend:
(a) eine Analysevorrichtung nach einem der Ansprüche 1 bis 16; und
(b) wenigstens ein Mittel zum Erzeugen eines Signals in wenigstens einer der genannten Reaktionszone und der genannten Kontrollzone.

41. Testsatz nach Anspruch 40, bei dem das Signale erzeugende Mittel an ein Element eines Paars von Ligand/Rezeptor konjugiert ist.

42. Testsatz nach Anspruch 40 oder 41, der weiter eine Probensammelvorrichtung aufweist.

## Revendications

1. Dispositif d'analyse pour déterminer la présence ou l'absence d'au moins un analyte dans un échantillon à tester de fluide obtenu d'un sujet à tester tout en fournissant une identification spécifique du sujet à tester, ledit dispositif comprenant un milieu de réaction, ledit milieu de réaction comprenant :
(a) au moins une zone de réaction qui comprend un élément d'une première paire de ligand/récepteur immobilisée sur celle-ci, ledit élément étant sélectionné de telle sorte que, lorsqu'un échantillon à tester suspecté de contenir un analyte spécifique entre en contact avec la zone de réaction, ledit élément fournit un moyen pour déterminer la présence ou l'absence dudit analyte, et
(b) au moins une zone de réaction qui comprend un élément d'une deuxième paire de ligand/récepteur immobilisée sur celle-ci, ledit élément étant sélectionné de telle sorte que, lorsqu'il entrera en contact avec un agent de production de signal, ledit élément réagira avec ledit agent de production de signal en vue de former un motif qui est capable d'établir l'identité d'un sujet à tester.

2. Dispositif d'analyse selon la revendication 1, dans lequel la zone de contrôle est capable de fournir un motif pour identifier le sujet à tester, dans lequel le motif comprend une empreinte digitale.

3. Dispositif d'analyse selon la revendication 1, dans lequel la zone de contrôle est capable de fournir un motif pour identifier le sujet à tester, dans lequel le motif comprend une empreinte de pied.

4. Dispositif d'analyse selon l'une quelconque des revendications précédentes, dans lequel ledit un élément d'une paire de ligand récepteur est capable de déterminer la présence ou l'absence d'au moins une des substances suivantes : cocaïne, benzoylecgonine, opiacés, phéncyclidine, amphétamine, méthamphétamine, tétrahydrocannabinol et alcool.

5. Dispositif d'analyse selon l'une quelconque des revendications précédentes, dans lequel la zone de réaction comporte un élément d'une paire immunologique.

6. Dispositif d'analyse selon l'une quelconque des revendications précédentes, dans lequel ledit élément de ladite deuxième paire de ligand/récepteur est un élément d'une paire immunologique.

7. Dispositif d'analyse selon l'une quelconque des revendications précédentes, dans lequel le milieu de réaction est une membrane poreuse.

8. Dispositif d'analyse selon l'une quelconque des revendications précédentes, dans lequel le milieu de réaction comprend plus d'une zone de réaction.

9. Dispositif d'analyse selon l'une quelconque des revendications précédentes, dans lequel l'agent de production de signal comprend un élément d'une paire de ligand/récepteur étiqueté avec un agent de formation de couleur.

10. Dispositif d'analyse selon l'une quelconque des revendications précédentes, dans lequel l'agent de production de signal comprend un agent de formation de couleur.

11. Dispositif d'analyse selon l'une quelconque des revendications précédentes, dans lequel l'échantillon à tester est un fluide biologique.

12. Dispositif d'analyse selon la revendication 11, dans lequel le fluide biologique est du sang, un produit du sang, de la salive ou de l'urine.

13. Dispositif d'analyse selon l'une quelconque des revendications précédentes, dans lequel ledit élément de ladite première paire de ligand/récepteur consiste en particules enrobées d'antigène ou d'anticorps.

14. Dispositif d'analyse selon l'une quelconque des revendications précédentes, dans lequel ledit élément de ladite première paire de ligand/récepteur consiste en particules enrobées d'anticorps.

15. Dispositif d'analyse selon l'une quelconque des revendications précédentes, qui comporte :
un support poreux ;
ledit milieu de réaction couvrant une partie du support poreux.

16. Dispositif d'analyse selon l'une quelconque des revendications précédentes, dans lequel la présence d'un analyte produit une couleur et l'absence d'un analyte ne produit pas de couleur.

17. Procédé pour tester la présence d'au moins un analyte dans un échantillon de fluide obtenu à partir d'un sujet à tester et identifiant positivement le sujet à tester, qui comprend :
(a) l'obtention d'un échantillon de fluide du sujet à tester ;
(b) l'application dudit échantillon de fluide à un dispositif d'analyse qui comprend un milieu de réaction, ledit milieu de réaction comprenant
(1) au moins une zone de réaction qui comprend un élément d'une première paire de ligand/récepteur immobilisée sur celle-ci, ledit élément étant sélectionné de telle sorte que, lorsque ledit échantillon à tester de fluide suspecté de contenir un analyte spécifique entre en contact avec la zone de réaction, ledit élément fournit un moyen pour déterminer la présence ou l'absence dudit analyte, et
(2) au moins une zone de contrôle pour établir l'identité dudit sujet à tester, ladite zone de contrôle comprenant un élément d'une deuxième paire de ligand/récepteur immobilisée sur celle-ci, ledit élément étant sélectionné de telle sorte que, lorsqu'il entrera en contact avec un agent de production de signal, ledit élément réagira avec ledit agent de production de signal en vue de former un motif qui établit l'identité dudit sujet à tester.
(c) l'enduisage d'un doigt ou de la plante d'un pied du sujet à tester avec un agent de production de signal et la mise en contact de la zone de contrôle du dispositif d'analyse avec ledit doigt ou pied enduit de telle sorte que ledit agent de production de signal réagisse avec ledit élément d'une deuxième paire de ligand/récepteur en vue de former un motif qui établit l'identité dudit sujet à tester ; et
(d) la détermination à partir de la zone de réaction si l'échantillon à tester de fluide contient ledit analyte spécifique.

18. Procédé selon la revendication 17, dans lequel la mise en contact du milieu de réaction avec l'échantillon à tester comprend l'application de l'échantillon à tester sur le milieu de réaction au moyen d'un applicateur.

19. Procédé selon la revendication 18, dans lequel l'applicateur comprend un doigt, un pied, un tampon ou une pipette.

20. Procédé selon l'une quelconque des revendications 17 à 19, dans lequel la formation du motif identifiant le sujet à tester comprend l'application d'un doigt du sujet à tester sur la zone de contrôle, produisant ainsi une empreinte digitale.

21. Procédé selon l'une quelconque des revendications 17 à 20, dans lequel l'établissement de l'identité de la personne comprend en outre l'enduisage du doigt de la personne avec un agent de production de signal avant l'application du doigt sur la zone de contrôle.

22. Procédé selon la revendication 21, dans lequel l'agent de production de signal comprend un élément d'une paire de ligand/récepteur étiqueté avec un agent de formation de couleur.

23. Procédé selon la revendication 21, dans lequel l'agent de production de signal comprend un agent de formation de couleur.

24. Procédé selon la revendication 23, dans lequel l'agent de formation de couleur comprend de l'or colloïdal.

25. Procédé selon la revendication 24, comprenant en outre la mise en contact de la zone de contrôle avec une solution d'enduisage d'argent après l'application du doigt sur la zone de contrôle.

26. Procédé selon l'une quelconque des revendications 17 à 25, dans lequel l'analyte est au moins une des substances suivantes : cocaïne, benzoylecgonine, opiacés, phéncyclidine, amphétamine, méthamphétamine, tétrahydrocannabinol et alcool.

27. Procédé selon l'une quelconque des revendications 17 à 26, dans lequel l'exécution de l'analyse de ligand/récepteur comporte la détermination de la présence ou de l'absence d'au moins un analyte dans un fluide biologique.

28. Procédé selon la revendication 27, dans lequel le fluide biologique est du sang, un produit du sang, de la salive ou de l'urine.

29. Procédé selon l'une quelconque des revendications 17 à 28, dans lequel l'exécution de l'analyse de ligand/récepteur comprend l'application de sang sur le milieu de réaction puis l'application d'une solution de clarification sur le milieu de réaction.

30. Procédé selon l'une quelconque des revendications 17 à 29, dans lequel l'exécution de l'analyse de ligand/récepteur comprend la détermination de la présence ou de l'absence d'une quantité seuil d'au moins un analyte dans l'échantillon à tester.

31. Procédé selon l'une quelconque des revendications 17 à 30, dans lequel la détermination de la présence de l'analyte produit une couleur dans une zone de réaction du milieu de réaction et la détermination de l'absence de l'analyte ne produit pas de couleur dans la zone de réaction.

32. Procédé selon l'une quelconque des revendications 17 à 31, dans lequel la détermination de la présence de l'analyte produit une couleur dans une zone de réaction du milieu de réaction et la détermination de l'absence de l'analyte ne produit pas de couleur dans la zone de réaction.

33. Procédé selon l'une quelconque des revendications 17 à 32, dans lequel l'étape c comporte la mise en contact directe de la zone de contrôle avec un applicateur étiqueté.

34. Procédé selon l'une quelconque des revendications 17 à 33, dans lequel ledit élément d'une première paire de ligand/récepteur comprend des particules enrobées d'anticorps ou d'antigènes.

35. Procédé selon la revendication 34, dans lequel ledit échantillon de fluide est combiné avec des anticorps anti-analyte avant d'être appliqué audit milieu de réaction, et ledit élément d'une première paire de ligand/récepteur comprend des antigènes qui se lient spécifiquement auxdits anticorps anti-analyte.

36. Procédé selon l'une quelconque des revendications 17 à 33, dans lequel ledit élément d'une première paire de ligand/récepteur comprend des anticorps anti-analyte.

37. Procédé pour tester la présence d'au moins un analyte dans un échantillon de fluide obtenu d'un sujet à tester et identifiant positivement le sujet à tester, qui comprend :
(a) l'obtention d'un échantillon de fluide suspecté de contenir un analyte spécifique provenant du sujet à tester et la combinaison de celui-ci avec des anticorps anti-analyte qui se lient spécifiquement audit analyte de manière à former une solution, par laquelle lesdits anticorps anti-analyte se lient spécifiquement audit analyte s'il est présent dans ledit échantillon de fluide ;
(b) l'application de ladite solution à un dispositif d'analyse qui comprend un milieu de réaction, ledit milieu de réaction comprenant
(1) au moins une zone de réaction qui comprend des antigènes immobilisés sur celle-ci, lesquels se lient spécifiquement auxdits anticorps anti-analyte, de telle sorte que, lorsque ladite solution entre en contact avec la zone de réaction les anticorps anti-analyte non liés dans ladite solution se lient auxdits antigènes immobilisés ;
(2) au moins une zone de contrôle pour enregistrer l'empreinte digitale ou l'empreinte de pied dudit sujet à tester, ladite zone de contrôle comprenant un anticorps anti-espèce immobilisé sur celle-ci, lequel se lie spécifiquement à un anticorps étiqueté ;
(c) l'enduisage d'un doigt ou de la plante d'un pied du sujet à tester avec un anticorps étiqueté qui se lie spécifiquement audit anticorps anti-espèce et audit anticorps anti-analyte ;
(d) la mise en contact de ladite zone de contrôle et de ladite zone de réaction du dispositif d'analyse avec ledit doigt ou pied enduit de manière à produire, respectivement, une empreinte digitale ou une empreinte de pied dans ladite zone de contrôle et à ce que ledit anticorps étiqueté se lie audit anticorps anti-analyte lié dans ladite zone à tester ;
dans lequel la présence dudit anticorps étiqueté dans ladite zone à tester indique l'absence desdits analytes dans ledit fluide corporel et l'absence dudit anticorps étiqueté dans ladite zone à tester indique la présence desdits analytes dans ledit fluide corporel.

38. Procédé pour traiter un échantillon de sang comprenant :
(a) la piqûre d'un doigt d'un sujet à tester en vue de former une gouttelette de sang ;
(b) le barbouillage du sang pour enduire le doigt d'une fine pellicule de sang ;
(c) l'application du doigt sur un milieu de réaction d'un dispositif d'analyse et le séchage du sang, dans laquelle ledit milieu de réaction est un milieu poreux qui comporte un élément d'une première paire de ligand/récepteur, lequel élément se lie spécifiquement à un analyte prédéterminé dans l'échantillon de sang ;
(d) l'application d'une solution de clarification audit milieu de réaction ;
(e) la détermination de la présence ou de l'absence d'au moins un analyte dans l'échantillon à tester ; et
(f) la formation d'un motif identifiant le sujet à tester dans une zone de contrôle du milieu de réaction, dans laquelle ladite zone de contrôle comporte un élément d'une deuxième paire de ligand/récepteur, ledit élément se lie spécifiquement à un agent de production de signal en vue de former un motif qui est capable d'établir l'identité du sujet à tester.

39. Procédé pour traiter un échantillon de sang selon la revendication 38, lequel comprend de plus l'application d'une solution de clarification au milieu de réaction avant d'appliquer le doigt.

40. Kit pour traiter un échantillon comprenant :
(a) un dispositif d'analyse selon l'une quelconque des revendications 1 à 16 ; et
(b) au moins un agent pour produire un signal dans au moins une de ladite zone de réaction et de ladite zone de contrôle.

41. Kit selon la revendication 40, dans lequel l'agent de production de signal est conjugué à un élément d'une paire de ligand/récepteur.

42. Kit selon la revendication 40 ou 41, comprenant en outre un dispositif de collecte d'échantillons.
